(19) Europäisches Patentamt
European Patent Office
Office européen des brevets

(11) Publication number: 0 537 722 A2

(12) **EUROPEAN PATENT APPLICATION**

(21) Application number: **92117569.1**

(22) Date of filing: **14.10.92**

(51) Int. Cl.[5]: **C12N 15/12**, C07K 13/00, C07K 15/00, A61K 37/02, A61K 35/407, //(A61K37/02, 31:505)

(30) Priority: **15.10.91 US 780084**
**04.12.91 US 803844**

(43) Date of publication of application:
**21.04.93 Bulletin 93/16**

(84) Designated Contracting States:
**AT BE CH DE DK ES FR GB GR IE IT LI LU MC NL PT SE**

(71) Applicant: **KREMERS- URBAN CO.**
**5600 West County Line Road**
**Mequon, Wisconsin 53092(US)**

(72) Inventor: **Wagle, Sudhakar S.**
**11505 North River Road**
**Mequon, Wisconsin(US)**
Inventor: **Tanaka, S. Ken**
**156 Copperwood Drive**
**Buffalo Grove, Illinois 60089(US)**
Inventor: **Steinbach, Thomas**
**No. 4 Bayou Shadows**
**Houston, Texas77024(US)**
Inventor: **Lawyer, Carl H.**
**10320 North Fontainebleu Court**
**Mequon, Wisconsin 53092(US)**
Inventor: **Hermann, William J., Jr.**
**103 River Ridge Road**
**Sealy, Texas 77474(US)**
Inventor: **Gawish, Ali Abdel Salam**
**11920 N. Lantern Lane**
**Mequon, Wisconsin 53092(US)**

(74) Representative: **von Füner, Alexander, Dr. et al**
**Patentanwälte v. Füner, Ebbinghaus, Finck**
**Mariahilfplatz 2 & 3**
**W-8000 München 90 (DE)**

(54) **Porcane liver polypeptides for treating viral infections.**

(57) The present invention is directed to substantially pure peptide composition having antiviral activity. These peptides are characterized by having a molecular weight of from between 5,000 to 40,000 Daltons. These peptides are further characterized by having at its 5' end the amino acids shown in Sequence Id. No. 1 and its 3' end either Sequence Id. Nos. 4 or 6. Additionally, this peptide is further characterized at its 5' end by being coded for DNA sequence shown in Sequence Id. No. 3. This composition can be used to treat viral infections, in particular HIV and HHV-6.

EP 0 537 722 A2

This application is a continuation-in-part of U.S. Serial No. 07/780,084 filed October 15, 1991 and U.S. Serial No. 07/804,844 filed December 4, 1991 which are a continuation-in-part of U.S. Serial No. 07/728,267 filed July 11, 1991, which in turn is a continuation of U.S. Serial No. 07/228,364 filed August 4, 1988, now U.S. Patent No. 5,055,296.

## FIELD OF THE INVENTION

The present invention is directed to a method and composition for treating viral infections in mammals and to the discovery that certain polypeptides originally derived from mammalian liver extract are efficacious in treating nondermatologic viral infections, in particular HIV infections.

## BACKGROUND OF THE INVENTION

Although recent decades have been marked by rapid advances in the treatment of bacterial infections, there have not been comparable advances in the treatment of viral infections. Indeed, today, very few efficacious antiviral agents exist.

Those that are known either suffer from restricted or limited efficacy or are toxic. For instance, amantidine is known to prevent influenza, but must be given prior to infection to be effective. Acyclovir, while often effective in treating herpes virus infections, selects for resistant strains of virus. Also, acyclovir is not effective against many kinds of viruses, including retroviruses and even some herpes viruses, such as cytomegalovirus. Other antiviral agents, such as 3'-azido-3'-deoxythymidine (AZT), are toxic, their activity relying upon a relative, but not absolute, selectivity for viral processes.

Indirectly-acting anti-viral therapies, including interferon and interferon inducers, enhance cellular responsiveness to viral infection, allowing the cells to interfere with the infection process. However, interferon has not fulfilled the promise of early expectations and theory. Interferon inducers are relatively new to the field and their efficacy remains unproven.

Acquired immune deficiency syndrome (AIDS) and AIDS-related complex (ARC) are caused by human immuno-deficiency virus (HIV-1), a retrovirus. The HIV-1 virus infects immune, neural and other cells of its host. Eventually most people infected with HIV-1 become abnormally susceptible to a variety of serious opportunistic diseases as a result of the immune deficiency caused by the virus.

The current anti-HIV-1 drugs are either not effective or cause undesirable side effects. These drugs include AZT, 2',3'-dideoxy cytidine (ddCyd), interferon (IFN), mismatched double-stranded RNA (dsRNA) and amphotericin B. In particular, AZT, which has shown some promise in the treatment of AIDS, causes very serious side effects, such as bone marrow suppression, in a high proportion of patients. Also, the beneficial effects of AZT have been reported to abate in 12-18 months, and patients get new infections or develop toxic side effects. Chase, "Doctors and Patients Hope AZT Will Help Stave Off AIDS." Wall Street Journal, April 28, 1988, at 14, col. 1.

Mammalian liver extract has been used for the treatment of a wide range of infectious and noninfectious dermatologic conditions, including acne vulgaris, Journal Invest Dermatology, 2:205-218 (1939); first and second degree burns, Mississippi Valley Medical Journey, 76:199 (1954); sunburn, Clinical Medicine, 3:245 (1956); poison ivy dermatitis, Clin. Med., 3:425 (1956) and Herpes zoster, Southern Medical Journal, 50:1524 (1957). The active principle and mechanism have not been described. Although some medical practitioners have used liver extract for the treatment of dermatologic conditions, it is not regarded as an antiviral or immune modulator agent even for skin therapy.

Mammalian liver extract has been reported to have bradykinin potentiating activity. Tewksbury et al., Arch. Biochem. Biophys. (U.S.), 112, 453 (1965); Tewksbury, Archives Int'l. de Pharmacodynamie et de Therapie, 173, 426 (1968); Tewksbury, Dissertation Abstracts International-Part II, Vol. 25/04, p. 2214 (1964). Further, one commercially-available liver extract (sold under the trademark KUTAPRESSIN by Kremers-Urban Co., Milwaukee, Wisconsin) exerts its action, according to product literature, only with respect to tissues that have been injured and when inflammation and edema are present.

The extract was found to be useful in treating chronic fatigue syndrome, See U.S. Patent No. 5,055,296, while certain purified peptides having a molecular weight of about 4,000 Daltons, were found to be useful in treating other viral infections such as HIV-1. These peptides required at least 39 $\mu$g/ml of purified peptide to protect against the virus. As such, a need existed to reevaluate the liver extract KUTAPRESSIN (Kremers-Urban Co.) to ascertain if potentially more physiologically active peptide fractions could be isolated from the liver extract.

## SUMMARY OF THE INVENTION

This invention is based on the discovery that certain substantially pure polypeptides having a molecular weight of between about 5,000-40,000 Daltons, obtained from an acetone - insoluble liver extract, when prepared in water-soluble form, are capable of inhibiting the replication and pathogenesis of the viruses which cause AIDS (e.g. AIDS-viruses), including both HIV-1 and HIV-2 viral subtypes. During experimental work on this invention, it was demonstrated that in vitro these water-soluble peptides can completely or partially protect human lymphocytes from infection by cell-free AIDS-viruses. This protective action is strongly positive at about a concentration of 12.5 µg/ml of active peptide, but is also significant at as little as 5 µg/ml of active peptide. Additionally, it was found that the active polypeptide functions synergisticly with AZT to suppress an AIDS infection.

## BRIEF DESCRIPTION OF THE DRAWING

Figure 1 illustrates the results of in vitro testing of the ability of a porcine liver extract to reduce the cytolysis of MT-2 lymphoblastoid cells upon infection with HIV-1 virus.

Figure 2 illustrates the results of in vitro testing of the ability of AZT, ampligen, castanospermine and two preparations of porcine liver extract to reduce the cytolysis of MT-2 lymphoblastoid cells upon infection with HIV-1 virus.

Figure 3 illustrates the results of in vitro testing of the ability of an active peptide to reduce the cytolysis of MT-2 lymphoblastoid cells upon infection with HIV-1 virus.

Figure 4 illustrates the results of in vitro testing of the ability of an active peptide to reduce the cytolysis of MT-2 lymphoblastoid cells upon infection with HIV-1 virus.

Figure 5 illustrates the results of in vitro testing of the ability of an active peptide to reduce the cytolysis of MT-2 lymphoblastoid cells upon infection with HIV-1 virus.

Figure 6 illustrates the results of in vitro testing of the ability of an active peptide to reduce the cytolysis of MT-2 lymphoblastoid cells upon infection with HIV-1 virus.

Figure 7 shows codon bias for HIV, using all pig sequences in Genbank.

Figure 8A & B show the strategy used to sequence the active polypeptide.

Figure 9 shows pH Gradient of Ku 10,260. This pH gradient was measured using a surface electrode for n = 4 gels run on or near the same date as the samples. The slab gel for the second dimension expands upon drying so that the final pattern is about 15.5 cm long. If SDS has been added to the sample, an SDS-NP40 micelle migrates to the extreme acid end of the tube gel, constricting the pH gradient. In this case the final 2-D pattern is 14.0-14.5 cm long. The black arrow on your 2-D gel indicates our internal standard, vitamin D dependent calcium binding protein (CaBP), pl 5.2 and M.Wt. 27,000. The M.Wt. standards are myosin (220,000), phosphorylase A(94,000), catalase (60,000), actin (43,000), and lysozyme (14,000) which have been added to the agarose.

Figure 10 shows serum p24 at the conclusion of the HIV/1 CEM mouse study.

Figure 11 shows synergy of AZT and Ku 10,004 when administered in vitro.

## Detailed Description of the Presently Preferred Embodiments

The portion of mammalian liver extract that has been discovered to be effective in treating viral infections is the fraction which is heat stable, insoluble in acetone and soluble in water. The liver extract prepared according to the disclosure herein is free from fatty acids, and vitamins, and specifically is free from vitamin B-12, a vitamin naturally occurring in liver. Work in progress suggests polysaccharides may be present in KUTAPRESSIN (Kremers-Urban Co.) in the form of proteoglycans and/or glycoproteins. The same liver extract has been used heretofore in treating skin conditions.

Additionally, the present invention provides certain polypeptides purified from an acetone-insoluble liver extract having anti-viral activity and being further characterized by a molecular weight in the range of about 5,000-40,000 Daltons. The polypeptides of this invention can also be identified by certain amino acid sequences. In particular, the polypeptide has at its 5' end the amino acid shown in Sequence Id. No. 1 and at its 3' end the amino acids shown in Sequences Id. Nos. 4 or 6. Additionally, the polypeptides of this invention can be characterized by amino acids at the 5' end that are coded for by DNA sequences shown in Sequence Id. No. 2 and being comprised of amino acids at the 3' end that are coded for by the DNA sequences show in Sequence Id. No. 3. Fragments of these polypeptides having anti-viral properties are also included within this invention. Additionally, synthetic polypeptides having the amino acid sequence shown in Sequence Id. Nos. 8-9 are also contemplated by this invention. This invention also provides a therapeutic method which involves administering to a mammal having a viral infection a therapeutically effective amount of the substantially pure polypeptide of this invention.

The DNA sequence of the active polypeptides were ascertained by determining the amino acid composition of the active peptides, preparing DNA fragments likely to hybridize to the gene encoding the active peptides and amplifying nucleic acid sequences that hybridize to these probes. Mullis, U.S. Patent No. 4,683,202 (Incorporated by Reference).

The activity of these polypeptides for inhibiting replication of HIV can be confirmed by known assays, including particularly, the assay described Montefiori et al., J. Clin. Micro. Biol. 231-235 (1968) Graham et al., Anti-Fusion Activity in Sera from Persons Infected with Human Immunodeficiency Virus Type 1, J. of Clin. MicroBio. 28:2608 (1990) (describes an antisyncytial assays using MT-2 cells) and Montefiori et al., Differential Inhibition of HIV-1 Cell Binding and HIV-1 Induced Syncytium Formation by Low Molecular Weight Sulphated Polysaccharides, J. Anti. Microb. Chemotherapy 25:315-318 (1990) (describes an antisyncytial and RT inhibition assay of III B isolates of HIV-1).

As determined by the MT-2 cell assay, effective in vitro inhibition levels are in the range from about 5 to greater than 500 micrograms ($\mu$g) per milliliter of solution. It is believed that effective in vivo levels will be similar. For example, desirable in vivo levels of active peptides in the blood are from about 5 to greater than 500 micrograms ($\mu$g) per milliliter of serum.

The active polypeptide treatments of this invention can be used with any patients infected with the HIV, including particularly viruses of the serological type HIV-1 but also including patients infected with other HIVs such as HIV-2. The treatment may be commenced with pre-AIDS apparently healthy persons infected with the virus. Persons manifesting the lymphadenopathy syndrome, or AIDS-Related Complex, may also be treated. Further, it is believed that the treatment will be of value for patients diagnosed as having AIDS. Preferably, however, it is believed that the treatment will be used with patients who have not progressed to active AIDS disease.

The effectiveness of the treatment can be monitored by known procedures and observations. For example, such procedures and observations were recently described: ASM News, Current Topics, 55:586-588, 1989. A laboratory test marker was recommended which involves observation of the level in the blood of CD4-bearing T lymphocyte cells. In patients that have the AIDS disease, the most direct criteria rest on clinical observations, such as a reduced incidence of opportunistic infections and extended lifespans. Other laboratory measures include tests for the p24 antigen of HIV, or additional components of the immune system including beta-2-microglobulin and/or neopterin. The criteria selected may depend on the stage of the HIV infection. For pre-AIDS patients, the quantity of HIV antigens detected in the blood may be the best measure. For example, Coulter Immunology Division of Coulter Corporation provides an HIV antigen Assay which can be used. This is an enzyme immunoassay using a murine monoclonal antibody (anti-HIV core antigen) coated onto microwell stripes. The assay detects HIV antigens in plasma or serum.

The scientific basis of the present invention is further elucidated by the following Experimental Examples.

## Preparation of the Liver Extract

The liver extract employed in the present invention is prepared by separating a fraction from mammalian livers, preferably porcine liver. The starting material may be a liver preparation as described in Pharmacopeia of the United States , Vol. 15, p. 379 (which describes a boiled liver extract suitable for parenteral use), in National Formulary, Vol. XII, p. 222 (which describes an aqueous solution of the thermostable fraction of mammalian liver) or in National Formulary, Vol. XI, p. 192-94 (which describes several thermostable liver preparations). Alternatively, the starting material may be fresh liver, frozen liver or a commercially-available liver preparation.

An acetone-insoluble fraction is separated from the starting material. This may be accomplished by admixing a large excess of acetone with the starting material which results in an acetone-insoluble fraction that is separated from the acetone. The treatment with acetone may be repeated. The acetone-insoluble fraction, after being separated from the acetone, is dissolved in water. Any remaining acetone is removed by, for example, distillation. The material effective in treating presenile or senile dementia is contained in the water solution.

Alternatively, and preferably, before the acetone extraction, the starting material is dissolved in water with phenol. The solution is incubated at room temperature and after incubation, the solution is clarified by filtration, and the solution is passed over a cation exchange resin. The resulting resin-treated solution is then concentrated by evaporation, diluted with water, and centrifuged. The acetone-insoluble fraction is then separated from the supernatant by adding a large excess of acetone and further processed as described above.

The acetone-insoluble fraction may be further purified to remove the color pigments by treatment with activated charcoal. For example, the acetone-insoluble fraction may be dissolved in water and contacted with ammonia-activated charcoal.

A pharmaceutically-acceptable preservative usually is added to the water solution. For instance, phenol at from about 0.05 to about 1%, preferably about 0.5% may be added.

The liver extract useful in the present invention may be prepared according to the following examples.

## Example 1 Liver Extract

Liver Fraction I, described in National Formulary XI, page 193, was dissolved in water to a concentration of 16% by weight. Phenol was added to a final concentration of 1%. The solution was mixed and incubated for seven days at room temperature. It was then clarified by filtration, and diluted to 8% solids by weight in water.

This aqueous solution was then passed three times through a cation exchange resin (sulfonated polystyrene). The resin-treated solution was clarified by filtration and concentrated to 40% total solids by weight by evaporation under vacuum at 65-70 °C. Cold water (5-10 °C) was added (five volumes of water to seven volumes of liver solution) with mixing. The resultant solution was then centrifuged and the supernatant collected (Sharples-type centrifuge at 1 liter per minute). Phenol was added to a final concentration 0.5-1%.

The solution was adjusted to pH 6.0-7.0, with HCl or NaOH as necessary, clarified by filtration, and heated to 40 °C. Then acetone was added (20-30 liters acetone per liter liver solution). The acetone-precipitable material was allowed to settle and most of the acetone was decanted off. The remaining suspension was incubated overnight at room temperature, after which the suspension was diluted to 10 liters with water, and the acetone was removed by distillation. Phenol and water were then added to give a final preparation containing 0.5% phenol and greater than 25 mg total solids per ml (herein designated "KU 10,000").

KU 10,000 was adjusted to pH 6.0-7.0 with HCl or NaOH, as necessary and diluted to 25 mg total solids per ml. with water (i.e., 2.5% by weight solids). The solution was then sterile filtered in suitable vials for use. This final solution is referred to herein as "KU 10,001".

## In Vitro Testing

KU 10,001 prepared according to Example 1 was tested using the microtiter infection assay system described in Journal of Clinical Microbiology, p. 231-235, Feb. 1968. Briefly, 100 microliters of KU 10,001 in growth medium (RPMI 1640 containing 16% fetal calf serum and 50 ug gentamicin per ml.) at various concentrations were added to the wells of a microtiter plate. MT-2 cells in 100 microliters of growth medium were added to give 3-4 x $10^4$ cells per well. The plates were incubated for 4 hours a 36 °C in 5% $CO_2$ in air and 100% humidity. Then 50 microliters of HIV-1 virus containing 5-25 x $10^4$ infectious particles were added to each well. The plates were incubated 3-5 days at 36 °C in 5% $CO_2$ in air and 100% humidity. The MT-2 lymphoblastoid cell line is a human T-cell lymphotropic virus I-transformed $T4^+$ T-lymphoblastoid cell line.

To assess culture viability and efficacy of treatment, 100 microliters of each test culture were then transferred to poly-L-lysine-coated wells in a new microtiter plate, and 100 microliters of 0.014% Finter neutral red in growth medium was added to each well. The plates were incubated or 1 hour a 36 °C, at which time the medium was removed, and the adhered cells were washed twice with 150 microliters of phosphate buffered saline. The dye was extracted from the adhered cells by adding 100 microliters of acidified alcohol (50% ethanol in 1% acetic acid), and the absorbance of the extracted dye solution at 540nm was measured.

The results of this assay are shown in Figure 1 which shows that KU 10,001 produced up to 42% cell protection from the cytotoxic effects of HIV-1 on the fourth day after HIV-1 was added to cultures of MT-2. This is a significant anti-HIV effect of the KU 10,001 liver extract and is comparable to that produced by other anti-HIV drugs such as AZT, ddCyd, rIFN-alpha, rIFN-beta, dsRNA, and amphotericin B.

Preliminary results indicated that the phenol at the concentrations present in the in vitro testing of KU 10,001 exhibited cytotoxic effects of the MT-2 cells and that the phenol had no significant antiviral activity. To assess whether this cytotoxic activity was obscuring the antiviral activity of KU 10,001, the following materials were prepared and tested in a microtiter infection assay.

1. KU 10,001 (prepared as described in Example 1).

2. KU 10,004-2 which was prepared by diluting KU 10,004-1 1:3 with water. KU 10,004-1 was prepared by extracting KU 10,000 twice with equal volumes of ethyl ether to remove the phenol. The Final aqueous phase is KU 10,004-1 which has a concentration of 80 mg. solids/ml. solution. Thus KU 10,004-2 has a concentration of 26.7 mg. solids per ml.

3. KU 10,006 which was prepared by precipitating KU 10,000 with 3.8 volumes of acetone to remove the phenol, recovering and drying the precipitate, and dissolving the precipitate in water at 55 mg solids/ml solution.

The results of the microtiter assay are shown in Table 1.

TABLE 1

| Dilution | % Cell Survival | | |
|---|---|---|---|
| | KU 10,001 | KU 10,004-2 | KU 10,006 |
| 1:40 | 14.0 | 55.6 | 48.7 |
| 1:80 | 32.7 | 73.8 | 82.0 |
| 1:160 | 30.6 | 77.4 | 105.7 |
| 1:320 | 14.2 | 30.1 | 111.8 |
| 1:640 | 0.3 | 7.4 | 100.8 |
| 1:1280 | -7.6 | 1.6 | 87.1 |
| 1:2560 | -12.4 | -2.0 | 27.2 |
| 1:5120 | -10.7 | 0.2 | 0.0 |

These results show that removal of the phenol substantially improved the ability of the MT-2 in vitro assay to detect the antiviral activity of liver extract.

A comparison was made of the activity of KU 10,004-1, KU 10,004-2, AZT, ampligen and castanospermine in a microtiter infection assay.

Ampligen $((rI_n)Xr(C_{12},U)_n)$ is a mispaired double-stranded RNA that triggers the induction of human interferon and has anti-HIV-1 activity. Montefiori and Mitchell, Proc. Nat'l Acad. Sci. U.S.A., 84(9), 2985-89-(May 1987).

Castanospermine has the following formula:It is extracted from the seeds of a leguminous tree, Castanospermun australe. Castanospermine also has anti-HIV-1 activity.

The various materials listed above were serially diluted and tested in the microtiter infection assay. The starting (undiluted) solution of each material had the following concentration:

Table 2

| Material | Concentration of Starting Solution (ug/ml) |
|---|---|
| AZT | 0.668 |
| Ampligen | 200 |
| Castanospermine | 118 |
| KU 10,004-1 | 3825 |
| KU 10,004-2 | 1275 |

The results of the assay of these materials in the microtiter infection assay are shown in Figure 2. As is apparent from Figure 2, KU 10,004-1 and KU 10,004-2 gave significant cell protection activity comparable to that of AZT and ampligen and better cell protection than castanospermine.

## ADMINISTRATION OF LIVER EXTRACT

The acetone-insoluble liver extract useful in the present invention preferably is administered by injection, for example, intramuscular injection. However, other forms of administration are contemplated.

The liver extract may be employed in the form of pharmaceutically-acceptable salts of the components, such as the alkali metal salts. The pharmaceutically-acceptable amides, lower alkyl esters, protected derivatives, other derivatives and analogues of the components of the liver extract are also contemplated.

Although, as indicated, the liver extract may be used as a water solution, it may also be utilized in association with other pharmaceutical carriers, or example, in saline solution. In any case, since the liver extract is preferably administered by injection, it is contemplated that the extract will be contained in a water base carrier. A preferred product is a water solution containing about 2.5% by weight of liver extract solids.

Dosages may vary depending upon the condition of the patient. Generally, however, it has been found that the administration of 2 ml of KU 10,001 prepared as described in Example 1 intramuscularly every other day will produce beneficial results in as little as about 4 weeks.

In Vivo Testing HIV 1 Infection

A 34 year old patient was found to have HIV antibody in August, 1988. The patient had no significant past medical illness, and, has since not experienced any medical problems including opportunistic infections. On May 29, 1990 the patient was begun and continued on treatment with KUTAPRESSIN (Kremers-Urban Co.) injection 2 cc intramuscular every day. The result of a test for T-lymphocyte function revealed a significantly suppressed response when his lymphocytes were incubated with the mitogen phytohemagglutinin (PHA) in a standardized in vitro tissue culture test system. The mitogen phytohemagglutinin (PHA) in a standardized in vitro tissue culture test system is a well recognized measure of the body's cellular immune competence essential in fighting the opportunistic infections which are the most frequent cause of death in HIV-1 infection (AIDS). The results are recorded below. A normal response is considered 50 times the baseline or unstimulated internal control. Following six months of receiving KUTAPRESSIN (Kremers-Urban Co.) the patient's T-lymphocyte response to PHA improved as indicated below:

Table 3

| | UNSTIMULATED BASELINE | PHA STIMULATED | BASELINE MULTIPLE |
|---|---|---|---|
| BEFORE KUTAPRESSIN (5/90) | 22 | 281 | 13 |
| AFTER KUTAPRESSIN (12/90) | 63 | 3867 | 61 |
| Normal | | | >50 |

This data demonstrates a significant improvement in lymphocyte function with KUTAPRESSIN (Kremers-Urban Co.) treatment for 6 months. It shows that although AZT alone inhibits the phytohemagglutinin (PHA) response (Munch-Petersen), when this patient on AZT received KUTAPRESSIN (Kremers-Urban Co.), his very abnormally depresses PHA baseline multiple [which suggests an increased risk of progression to AIDS (Cunningham-Rundles)] improved to within the normal range [which suggests a lessened risk of progression to AIDS due to the anti-HIV-1 effects of KUTAPRESSIN (Kremers-Urban Co.) treatment].

In Vivo Testing HIV-1 Infection

This case of a 50 year old white male, who completely asymptomatic in 1986, underwent a routine HIV test and on December 15, 1986 he was found to be HIV positive with a T-4 cell count of 780. In September of 1989 he was started on AZT taking six capsules a day because his T-4 cell count had dropped to a level of 180, at that time he was experiencing some fatigue and a mild skin problem which was later diagnosed as eosinophilic folliculitis. He was also started on Pentamidene IPPB on a monthly basis. He continued to work at his regular job as the chief administrator of a nursing facility. In January of 1990 with a T-4 cell count of 100 he was started on KUTAPRESSIN (Kremers-Urban Co.) 2cc's IM every Monday, Wednesday and Friday and has continued this dosage over the years until the present time. In January of 1990 his only symptoms were mild fatigue and the skin problem with the eosinophilic folliculitis which resulted in minor itching of the skin. In May of 1991 his T-4 cell count had dropped to 36. He was still not ill except minor episodes of fatigue and mild problems with the skin infection. This was treated just symptomatically and he continued to work everyday as administrator of a nursing facility He was started on DDC in May of 1991 taking .735mg three times daily. The AZT was discontinued at this time and the KUTAPRESSIN (Kremers-Urban Co.) injections continued at a level of 2cc's IM every Monday, Wednesday and Friday. The patient continued on this program and remains on this program at this time. In June of 1991 his T-8 suppressors numbered 1800 and the T-4 cells numbered 36. The patient continued on the therapy as outlined from May 1991 and in July of 1992 the following figures were available; ten T-cells (3 recorded at 63.4% or 1,593), the T-Helper or T-4 cells were .03% or 8, the T-Suppressor or T-8 cells numbered 63.2% or 1,588. The patient's only symptoms were minor fatigue and the continuing problem with the skin characterized by itching and mild folliculitis and the diagnosis again after seeing several dermatologists is still eosinophilic folliculitis. He continues to work everyday and has never been hospitalized, he has never suffered from any significant one of the usual AIDS diagnoses and would not really be characterized as an AIDS patient if his T-cell count were above 200. His medications continue to be Pentamidene IPPB every month, DDC .375mg three times daily, KUTAPRESSIN (Kremers-Urban Co.) 2cc's IM every Monday, Wednesday and Friday. The patient takes vitamins daily and uses a cream for this skin problem. I have recently examined this patient and find him to be in fairly good health. His p-24 test has remained negative the entire time that he has been treated with KUTAPRESSIN (Kremers-Urban Co.).

In Vitro Testing Human Herpesvirus-6

In vitro testing in $HSB_2$ cells show the effect of KUTAPRESSIN (Kremers-Urban Co.) on the infection of HHV-6 (GS). HHV-6 is human herpesvirus-6. This virus was first isolated from AIDS patients and patients with other lymphoproliferative disorders. Ablashi, Human B-Lymphotropic virus (human herpesvirus-6), J. Virol. Met. 21:29-48 (1988).

$HSB_2$ cells were pretreated with 100, 300 and 500 $\mu$L Levels of the KUTAPRESSIN (Kremers-Urban Co.) for 24 hours. The KUTAPRESSIN (Kremers-Urban Co.) was preapred as designated in Example 1. Cells were washed and infected with HHV-6 (GS isolate) using approximately 1000 $TCID_{50}$/ml for two hours and KUTAPRESSIN (Kremers-Urban Co.) was added to the cells after removing the undesirably virus. The cells were examined for viability, morphological changes (Giant cell formation) and antigen expression by IFA using a polyclonal human antibody and serum to HHV-6 (N901) and two HHV-6 monoclonal antibodies. (D12 and 5CEA) A summary of the results follows:

Cells were treated with the drug for 24 hours. The drug was then removed, and cells were infected with 1,000 TCID50 of HHV-6 was adsorbed for two hrs. Unadsorbed virus was removed, and cells were fed with medium containing the drug. The cells were examined for infection by indirect immunofluorescence assay, using HHV-6 antibody positive human serum (N901).

Table 4

A. Days post infections

| Days post infections | 300µg(%+) | 500µg(%+) |
| --- | --- | --- |
| 3 | - | - |
| 7 | 20 | 5 |

B. Treatment of cells with the drug for 24hrs. After virus adsorption cells were <u>not</u> kept in the drug.

| Days post infection | 300µg(%+) | 500µg(%+) |
| --- | --- | --- |
| 3 | <3 | <1 |
| 7 | 80 | 80 |

Treated the cells with 1,000 TCID50 of the virus for 2hrs., removed the unadsorbed virus, and then kept the cells with the drug.

A.

| Days post infection | 300µg(%+) | 500µg(%+) |
| --- | --- | --- |
| 3 | - | - |
| 7 | 10-20 | ≤1 |

Virus and drug added to the cells at the same time.

B.

| Days post infection | 300µg(%+) | 500µg(%+) |
| --- | --- | --- |
| 3 | - | - |
| 7 | <1 | - |

Virus alone, no drug treatment.

C.

| Days post infection | 300µg(%+) | 500µg(%+) |
| --- | --- | --- |
| 3 | 2 | <1 |
| 7 | >80 | >80 |

No virus, only drug-treated cells.

D.

| Days post infection | 300µg(%+)* | 500µg(%+)* |
| --- | --- | --- |
| 3 | - | - |
| 7 | - | - |

*>81% of cells were viable.

The more detailed results of these tests are shown in Table 5 to 8.

Table 5

| Treatment ment | Days post treatment | Cell viability (%) | Cell Count | HHV-6 antigen positive cells (%) N901, | D12 | 5CEA |
|---|---|---|---|---|---|---|
| 100μg | 3 | 76.6 | $9.8 \times 10^5$ | 5 | 10 | 12 |
| | 14 | 73.7 | $1.23 \times 10^6$ | 90 | 82 | 68 |
| 300μg | 3 | 74.1 | $1.06 \times 10^6$ | 0 | 0 | 0 |
| | 14 | 76.1 | $1.18 \times 10^6$ | 3 | 1 | 1 |
| 500μg | 3 | 72.6 | $1.7 \times 10^6$ | 0 | 0 | 0 |
| | 14 | 73.8 | $1.07 \times 10^6$ | <1 | <1 | <1 |

<1 = very few cells antigen positive

| | | | | | | |
|---|---|---|---|---|---|---|
| HHV-6 | 3 | 78.2 | $4.2 \times 10^5$ | 10 | 16 | 18 |
| Only | 14 | 63.5 | $5.8 \times 10^6$ | 90 | 90 | 88 |
| Only drug | 3 | 92.2 | $2.93 \times 10^6$ | 0 | 0 | 0 |
| 300μg | 14 | 81.0 | $2.2 \times 10^6$ | 0 | 0 | 0 |
| 500μg | 3 | 93.2 | $2.7 \times 10^6$ | 0 | 0 | 0 |
| | 14 | 90.5 | $2.45 \times 10^6$ | 0 | 0 | 0 |

Table 6

In Vitro Effect of KUTAPRESSIN on HHV-6

The treatment was the same as in Table 5 except after virus adsorption the cells were kept in tissue culture medium.

| Treatment ment | Days post treatment | Cell viability (%) | Cell Count | HHV-6 antigen positive cells (%) N901, | D12 | 5CEA |
|---|---|---|---|---|---|---|
| 100μg | 3 | 78.5 | $2 \times 10^6$ | 5 | 8 | 7 |
| | 14 | 71.2 | $7.8 \times 10^5$ | 90 | 88 | 91 |
| 300μ | 3 | 78.6 | $8.8 \times 10^5$ | 4 | 8 | 6 |
| | 14 | 69.4 | $7.7 \times 10^5$ | 90 | 80 | 85 |
| 500μ | 3 | 76.0 | $1.3 \times 10^6$ | 4 | 6 | 5 |
| | 14 | 72.8 | $1.07 \times 10^6$ | 70 | 75 | 71 |
| HHV-6 | 3 | 78.2 | $4.2 \times 10^5$ | 10 | 16 | 18 |
| Only | 14 | 63.5 | $5.8 \times 10^6$ | 90 | 90 | 88 |
| Only drug | 3 | 92.2 | $2.93 \times 10^6$ | 0 | 0 | 0 |
| 300μg | 14 | 81.0 | $2.2 \times 10^6$ | 0 | 0 | 0 |
| 500μg | 3 | 93.2 | $2.7 \times 10^6$ | 0 | 0 | 0 |
| | 14 | 90.5 | $2.45 \times 10^6$ | 0 | 0 | 0 |

Table 7

In Vitro Effect of KUTAPRESSIN on HHV-6

$HSB_2$ cells were first infected with the virus and drug the same time and kept together.

| Treatment ment | Days post treatment | Cell viability (%) | Cell Count | HHV-6 antigen positive cells (%) N901, | D12 | 5CEA |
|---|---|---|---|---|---|---|
| 100μg | 3 | 81.8 | $3.6 \times 10^5$ | 3 | 5 | 8 |
| | 14 | 73.0 | $1.7 \times 10^6$ | 50 | 48 | 46 |
| 300μ | 3 | 83.4 | $3.8 \times 10^5$ | <2 | <2 | <2 |
| | 14 | 73.8 | $1.17 \times 10^6$ | 8 | 8 | 10 |
| 500μ | 3 | 80.1 | $2.5 \times 10^5$ | <1 | <1 | <1 |
| | 14 | 72.0 | $1.05 \times 10^5$ | 2-3 | 3 | 4 |
| Virus | 3 | 78.8 | $4.5 \times 10^6$ | 6 | 8 | 10 |
| Only | 14 | 60.0 | $5.8 \times 10^6$ | 91 | 96 | 88 |
| Only drug | 3 | 92.2 | $2.93 \times 10^6$ | 0 | 0 | 0 |
| 300μg | 14 | 81.0 | $2.2 \times 10^6$ | 0 | 0 | 0 |
| 500μg | 3 | 93.2 | $2.7 \times 10^6$ | 0 | 0 | 0 |
| | 14 | 90.5 | $2.45 \times 10^6$ | 0 | 0 | 0 |

Table 8

In Vitro Effect of KUTAPRESSIN on HHV-6

$HSB_2$ were infected for three days with HHV-6 (GS) and then drugs was added to the cell culture.

| Treatment ment | Days post treatment | Cell viability (%) | Cell Count | HHV-6 antigen positive cells (%) N901, | D12 | 5CEA |
|---|---|---|---|---|---|---|
| 100μg | 3 | 81.4 | $3.5 \times 10^5$ | 12 | 18 | 20 |
| | 14 | 70.3 | $8.8 \times 10^5$ | 80 | 90 | 90 |
| 300μg | 3 | 87.8 | $3.6 \times 10^5$ | 6 | 8 | 10 |
| | 14 | 71.2 | $8.8 \times 10^5$ | 75 | 78 | 81 |
| 500μg | 3 | 84.3 | $3.3 \times 10^5$ | 5 | 7 | 10 |
| | 14 | 72.0 | $1.8 \times 10^5$ | 60 | 65 | 63 |
| HHV-6 | 3 | 76.2 | $3.2 \times 10^6$ | 22 | 30 | 29 |
| Only | 14 | 58.5 | $4.2 \times 10^5$ | 91 | 88 | 92 |
| Only drug | 3 | 92.2 | $2.93 \times 10^6$ | 0 | 0 | 0 |
| 300μg | 14 | 81.0 | $2.2 \times 10^6$ | 0 | 0 | 0 |
| 500μg | 3 | 93.2 | $2.7 \times 10^6$ | 0 | 0 | 0 |
| | 14 | 90.5 | $2.45 \times 10^6$ | 0 | 0 | 0 |

The results of these experiments show that KUTAPRESSIN (Kremers-Urban Co.) is effective in reducing HHV-6 antigen. The most effective treatment in these experiments is pretreatment with KUTAPRESSIN (Kremers-Urban Co.) and then after viral adsorption to continue to culture with KUTAPRESSIN (Kremers-Urban Co.).

In Vivo Testing of KUTAPRESSIN on Patients with CFS

A group of 131 patients suffering from CFS were treated with KU 10,001 prepared as described in Example 1. The patients used in the study were diagnosed as having CFS because they satisfied the criteria described in Ann. Int. Medicine, 108:387 (1988).

Before beginning the treatment, numerous laboratory tests were performed including a test for IgG antibodies to Epstein-Barr virus early antigen (EA). The presence of antibodies to EA indicates the presence of an active Epstein-Barr infection. The number of patients in the study group testing positive (i.e., had a titer of 1:80 or greater) for antibodies to EA are shown in Table 9.

The patients were injected intramuscularly with 2 ml. of KU 10,001 every other day. The total number of infections given to each patient varied.

The patients were asked to rate their improvement as either: 1) no improvement; 2) slight improvement; 3) moderate improvement; 4) notable improvement; or 5) marked improvement. The results of these ratings are given in Table 9. Table 9 also gives the average total number of injections given to each group of patients.

TABLE 9

| Rating | Number of Patients | Number of Patients Positive For EA | Average Total Number of Injections |
|---|---|---|---|
| No improvement | 8 | 6 | 11.0 |
| Slight improvement | 11 | 8 | 10.9 |
| Moderate improvement | 19 | 17 | 19.0 |
| Notable improvement | 39 | 21 | 18.1 |
| Marked improvement | 54 | 36 | 16.5 |

As shown in Table 9, treatment of patients diagnosed as having CFS with KU 10,001 resulted in a least a slight improvement in the condition of 94% of the patients. A very substantial number of patients (71%) showed notable or marked improvement.

The above disclosure sets forth what the inventors believe is a unique and hitherto unknown method of treating mammals, including humans, infected with virus or suffering from CFS by using a liver extract. The action of the liver extract may be the result of direct virucidal or virustatic activity or may be the result of modulation of the immune response.

The KU 10,001 liver extract of Example I has a long history demonstrating lack of toxicity, and a long clinical experience in treating dermatologic conditions. These constitute a distinct advantage over other anti-virus drugs, many of which suffer from very serious toxicity.

Example II -- Physically Active Polypentide

A large excess of acetone "1800 ml" was added to eight vials KU 10,001 prepared according to Example I, 20 ml/vial, total of 160 ml, and left to stand at room temperature for four hours. After the precipitate settle down at the bottom of the beaker, the clear acetone layer decanted and the remaining suspension centrifuged for five minutes at 3000 RPM. The pellet then dissolved in 160 ml of water and freeze dried to produce about 4.0 g of dry powder consists of 0.3 mg protein/1 mg of dry powder weight. These samples were designated KU 10,172, KU 10,185, KU 10,211, KU 10,244 and KU 10,275

One gram of the dry powder was taken in 7 ml of 50 mM phosphate buffer, pH 7.5 and passed through a 100 x 2.5 cm column packed with Sephadex G50 suitable for use as a molecular sieve that exclude (does not retard) molecules with a molecular weight greater than 30,000 or BIOGEL p10 which exclude molecules with a molecular weight greater than 20,000. The column was equilibrated with 50 mM phosphate buffer before use at flow rate of 36 ml/hr. The column was eluted with 50mM phosphate buffer pH 7.5. Seven ml. fractions were collected, and read at A280. Fractions were tested for angeotensin converting enzyme inhibition (using Furylacryloylphenylalanylglycylglycine as substrate) as described by Bush, Henry and Slasarchyk; J. of Antibiotics 37(4), 330 (1984). All fractions eluted before angeotensin converting enzyme inhibition were pooled according to the following table.

Table 10

| Loaded KU# | KU# | Pool# | Tube # |
|---|---|---|---|
| 10,244 | 10,245 | 1 | 22-30 |
| | 10,246 | 2 | 31-50 |
| 10,185 | 10,190 | 1 | 14-16 |
| | 10,191 | 2 | 18-20 |
| | 10,192 | 3 | 21-23 |
| | 10,193 | 4 | 26-29 |
| 10,275 | 10,275-I | 1 | 35-46 |
| | 10,275-II | 2 | 47-58 |
| | 10,275-III | 3 | 59-68 |

All pooled samples were concentrated, dialyzed in cellulose dialysis tubing with a molecular weight cut off of 1,000 and lyophilized.

Ku 10,275 was further purified by running the compound over large G-50 (Pharmacia Co.) column, desalted with amicon filter with a molecular weight of Cut off of 3500. The polypeptide was lyophilized. The molecular weight was determiend to be about 10,000 daltons and a protein content of .72% was determined. This polypeptide was designated Ku 10,280.

In Vitro Testing

Polypeptides prepared according to Example II were tested using the microtiter infection assay system described in Journal of Clinical Microbiology, p. 231-235, Feb. 1968. Briefly, 100 microliters of KU 10,001 in growth medium (RPMI 1640 containing 16% fetal calf serum and 50 µg gentamicin per cl.) at various concentrations were added to the wells of amicrotiter plate. MT-2 cells in 100 microliters of growth medium were added to give 3-4 x $10^4$ cells per well. The plates were inacubated for 4 hours at 36°C in 5% $CO_2$ in air and 100% humidity. Then 50 microliters of HIV-1 virus containing 5-25 x $10^4$ infectious particles were added to each well. The plates were incubated 3-5 days at 36°C in 5% $CO_2$ in air and 100% humidity. The MT-2 lymphoblastoid cell line is a human T-cell lymphotropic virus I-transformed $T4^+$ T-lymphoblastoid cell line.

To assess culture viability and efficacy of treatment, 100 microliters of each test culture were then transferred by poly-L-lysine-coated wells in a new microtiter plate, and 100 miroliters of 0.014% Finter neutral red in growth medium was added to each well. The plates were incubated for 1 hour at 36°C, at which time the medium was removed, and the adhered cells were washed twice with 150 microliters of phosphate buffered saline. The dye was extracted from the adhered cells by adding 100 microliters of acidified alcohol (50% ethanol in 1% acetic acid), and the absorbance of the extracted dye solution at 540nm was measured.

The results of this assay are shown in Figures 3-5 which shows that the active peptide produced nearly 100% cell protection from the cytotoxic effects of HIV-1 after HIV-1 was added to cultures of MT-2 at approximately 8 µg/ml. Additionally, 50-60% activity was observed on the average at as little as 4 µg/ml. This is a significant improvement over KU-10,001 liver extract where 50% activity was observed at 39 µg/ml concentration of active peptide.

Further Purification of Physically Active Polypeptide

KU 10,172 prepared according to Example II was fractionated on reverse phase $C_{18}$ prep column, eluted with buffer A: 20mM ammonium acetate pH 7.0, B: 80% acetonitrite in buffer A, gradient run at 214nm, programmed: zero to 80% B in 80 min. at 8.4 ml/min. Fractions collected 8.4 ml/test tube. All tubes were analyzed by analytical $C_{18}$ reverse phase column and size exclusion high pressure liquid chromatography column Tsk 125 and pooled in to twelve fractions based on retention times. Eight fractions KU 10,201 to KU 10,208 were tested for anti-viral activity and showed a significant cell protection activity. See Fig. 6.

Additionally, KU 10,203 and KU 10,207 prepared according to Example III were further purified on reverse phase $C_{18}$ prep column, eluted with buffer A: 20 mM ammonium actuate pH 7.0, B: 80% acentonitrite in buffer A, gradient run at 214 nm, programmed: Zero to 80% B in 80 min, at 8.4 ml/min. Fractions collected 8.4 ml test tube. All tubes were analyzed by analytical $C_{18}$ reverse phase column and size exclusion high pressure liquid chromatography column (Tsk 125) and pooled according to its retention times to produce KU 10,214 and KU,215. The cDNA from rat liver for the KU 10,214 and KU 10,215 fractions that were active in the bioassay was isolated and cloned using the polymerase chain reaction technique. The desired sequence to be amplified was that of the gene in pig liver cells that encodes the peptides in the KU 10,214 and KU 10,215 fractions.

Active Fraction Peptide Amino Terminal Amino Acid Sequencing

Ten amino acids of amino terminal sequence of KU 10,214 and KU 10,215 were determined by Edman degradation using an Applied Biosystems model 477A automated peptide sequencer with attached High Pressure Liquid Chromatography model 120A on-line phenyl isothiocyanate analyzer found to be (Ala or Val or Ile) Glu (His or Pro) Gly (Tyr or Met or Thr) His Gly Pro His Gly. Sequence Id. No. 10. More specifically, KU 10,214 has the amino acid sequence:
(Ala or Val or Ile) - (Glu or Gln) - (His or Pro or Arg) - Gly - Thr - His - Xaa - pro - His - Gly. Sequence Id. No. 11.
KU 10,215 has the amino acid squence:
(Ala or val or Ie) - (Glu or Gln) - (His or Pro) - Gly - (Tyr or Met) - His - Gly - Xaa - His - Gly - Xaa - Xaa - Gly -Xaa- Gln. Sequence Id. No. 12.
Due to the similarity of both sequences, the following deca peptide sequence, Ala - Glu - His - Gly - Tyr - His - Gly - Pro - His - Gly, Sequence Id. No. 13, was used in the Polymerase Chain Reaction Work.

Polymerase Chain Reaction Primer Design

The oligonucleotide primer 5'CATGGICCICATGGI3' [I indicates Inosine] (Sequence Id. No. 14) was designed based on the five amino acid sequence (HGPHG) region sequence that was common to both the KU 10,214 and KU 10,215 fractions active in the bioassay. This primer corresponds to the indicated amino acids regardless of codon usage except for His. Codon bias analysis for all pig gene sequences found in the databank 66 showed His codon at CAT to be used about 2.5 times as often as His codon CAC. This and the rarity of CG dinucleotides in peptide coding regions of mammalian genomes (Sambrook, J., Fritsch, E.F. and Maniatis, T. (1989) in "Molecular Cloning: A Laboratory Manual", 2nd edition, Cold Spring Harbor Laboratory, Cold Spring Harbor, New York) dictated use of CAT and not CAC for His in this sequence producing a primer sequence of 5'CATGGICCICATGGI 3'. Sequence Id. No. 14. This primer was prepared by conventional techniques using an Applied Biosystems DNA synthesizer. See Fig. 5.

RNA Isolation

Total RNA (2.5 mg) was isolated from 1.5 grams of fresh liver tissue from a female pig by rapid homogenation in guanidine thiocyanate followed by extraction with phenol (Chomczynski, P. and Sacchi, N. (1987) Anal. Biochem 162, 156-159). After two successive precipitations with isopropanol, the purified RNA was dissolved in water. The RNA was then subjected to poly(U) Sepharose chromatography, (Jacobson, A. (1987) Meth. Enzymology 152, 254-261), which yielded 21 µg of Poly(A)+RNA from 1 mg of total RNA.

cDNA Synthesis and Intermediate Polymerase Chain Reaction Amplification

Double stranded cDNA was prepared from the Poly(A)+RNA by a modification of the method of Gubler and Hoffman (Gene 25, 283 (1983)). Five 5µg of Poly(A)+RNA was used with an oligo(dT)-Hind III primer and AMV reverse transcriptase to synthesize the first strand, and second strand conversion was accomplished using RNase H and E. Coli DNA polymerase I. The yield was 30% first strand conversion and 100% second strand conversion. The double stranded cDNA was extracted with phenol and precipitated with ethanol by conventional methods. After treatment with T4 DNA polymerase to flush the ends, the cDNA was ligated with UNIAMP adaptors (Clontech Labs Inc. 800-662-CLON) under conditions described by the manufacturer. Following ligation, 3 µl of a 1:10 diluation of the cDNA was amplified by polymerase chain reaction in the Perkin Elmer Cetus GENEAMP System using a single UNIAMP primer (Clontech) under conditions described by the manufacturer. A sample of the reaction products was analyzed by agarose gel electrophoresis in the presence of ethidium bromide. The results showed a distribution of cDNA products that closely matched the pattern of unamplified cDNA. The remainder of the reaction products were purified by extraction with phenol and SEPHAROSE (Pharmacia Co.) CL-4B chromatography. These intermediate amplification and purification steps produced a sufficient amount of pure cDNA free of extraneous sequences which could have interfered with subsequent procedures.

Polymerase Chain Reaction Amplification with the Specific primer

The amplified cDNA was precipitated with ethanol, collected by centrifugation and dissolved in 20 µl water. A 1 µl sample was used for polymerase chain reaction amplification with the specific primer described above in combination with oligo (dT)-Hind III primer. Conditions for polymerase chain reaction were 30 cycles at 94°C for 1 minute, and 72°C for two minutes per cycle, with a final extension at 72°C for five minutes. A fraction of the reaction products were analyzed by agarose gel electrophoresis in the presence of ethidium bromide. The results showed three major DNA species in the range of 200 bp (base pairs), 400 base pairs and 500 base pairs. Control reactions in which the primers were omitted produced no detectable products. The strategy for sequencing this DNA is shown in Figure 7.

Cloning of the Polymerase Chain Reaction products

The remainder of the polymerase chain reaction products was treated with T4 DNA polymerase to flush the ends, and then purified by phenol extraction and ethanol precipitation. The DNA was ligated with ECORI linkers under standard conditions. Following digestion with ECORI and Hind III and removal of small molecules by SEPHAROSE (Pharmacia Co.) CL-4B chromatography, the prepared DNA was ligated with ECO RI/Hind III EXLOX vector arms, packaged in vitro, and plated on E. Coli by standard methods. The resulting library contained 3 x $10^5$ independent clones and was amplified to a titer of 3 x $10^{10}$ pf$\mu$/ml.

Verification of cloning

The library was plated at a density corresponding to approximately 1,000 plaques per 82 mm plate. Plaque lifts were prepared and hybridized with random-primer labeled DNA probes by conventional methods. Using a probe from DNA amplified with the specific primer described above, virtually every plaque produced a positive hybridization signal. This indicated the library contained the desired inserts of polymerase chain reaction products.

DNA sequencing

Six randomly chosen plaques were converted to plasmid subclones for DNA sequence analysis. Restriction enzyme analysis showed an insert of approximately 500 base pairs in four of these isolates. Plasmid DNA was prepared and sequenced directly using T7 DNA polymerase (promega) and chain-terminating dideoxynucleotides (Mierendorf, R.C. and Pfeffer, D. (1987) Meth. Enzymol. 152, 556-562).

DNA Sequencing

Sequencing experiments determined 96 base pairs at the 5' end and 110 pairs at the 3' of the cDNA insert, which appeared to be identical in the clones sequenced.

The 5' 96 base pairs encoded a 32 amino acid polypeptide (Sequence Id. No. 1) whose first four amino acids GPHG corresponded to those of the primer HPGHG. (Sequence Id. No. 15). (Sequence Id. No. 2) The 110 pairs at the 3' end (Sequence Id. No. 3) had a TAA or ATG stop codon in all three reading frames, so the C-terminal of the polypeptide is L (encoded by 5'CTA3'), (Sequence Id. No. 4 which has 8 amino acids and is encoded by (Sequence Id. No. 5) and/or (Sequence Id. No. 6) which has 21 amino acids and is encoded by Sequence Id. No. 7. As such the polypeptides are characterized by the Sequence Id. No. 1 at the 5' end and Sequence Id. Nos. 4 and 6.

Example V Physical and Chemical Tests on Physiologically Active Polypeptide

Thus, the physiologically-active polypeptide may be characterized by its physical and chemical properties. The active polypeptide is insoluble in acetone, and soluble in water. It has a molecular weight as determined by molecular sieve chromatography experiments to be about 5,000 - 40,000. The physiologically active peptide is acidic having the pH gradient shown in figure 8.

ADMINISTRATION OF POLYPEPTIDES

The polypeptides useful in the present invention preferably are administered by injection, for example, intramuscular injection. However, other forms of administration are contemplated. The polypeptides may be employed in the form of pharmaceutically-acceptable salts of the components, such as the alkali metal salts. The pharmaceutically-acceptable amides, lower alkyl esters, protected derivatives, other derivatives and analogues of the components of the polypeptides are also contemplated.

Although, as indicated, the polypeptides may be used as a water solution, it may also be utilized in association with other pharmaceutical carriers, for example, in saline solution. In any case, since the polypeptide is preferably administered by injection, it is contemplated that the extract will be contained in a water base carrier. A preferred product is a polypeptide water solution containing about 2.5% by weight of polypeptide. More generally, the polypeptide ranges from 5 $\mu$g to 500 $\mu$g per ml of carrier.

HIV-1/CEM Mouse Study

Nude mice transplanted with HIV infected cells were used to evaluate KU 10,280 for in vivo anti-HIV efficacy. See Wetherall, The Development of HIV-1 p. 24 Antigenemia in the Athymic "Nude Mouse" Animal Models in AIDS 291 (1990 Elevier Science Publishers B.V. hereby incorporated by reference). In this test eight irradiated nude mice transplanted with HIV infected CEM cells were used to determine whether KU 10,280 can effectively inhibit HIV within this model system. The drug AZT, which was previously shown to be effective, was used as the control. See Li et al., The Nude Mouse Transplanted With HIV Infected Cells as a Model for the evaluation of antivirals, Amer. Soc. Microbiol. Abstract Number 230060 (1992).

Experimental Methods:

Cell Line and HIV Culture. CCRF-CEM or CEM is a well characterized (Foley GE, Lazarus H, Farber S, Uzman BG, Boone BA, and McCarthy Re: Continuous culture of human lymphoblasts from peripheral blood of a child with acute leukemia. Cancer 1965;18:255-529 tumorigenic (Graham BS, and Wetherall NT: Growth of human cell lines in BALB/c mice. Cancer Res 1990;50:5943-5946) and HIV permissive (Dalgeish aG, Beverly PCL, Clapham PR, Crawford DH, Greaves MF, and WEiss RA: The CD4(T4) antigen is an essential component of the receptor for the AIDS retrovirus. Nature 1984;312:736-767) cell line, was acquired from the American Type Culture Collection, Rockville, MD. (ATCC CCL 119) and maintained in RPMI 1640 medium with 15% heat inactivated fetal calf serum and 50 µg/ml gentamicin as described previously. (Wetherall NT: The development of HIV-1 p24 antigenemia in the athymic "nude mouse." In: Animal Models in AIDS. Schellekens H and Horzinek MC (eds.). Elsevier; Amsterdam, 1990, pp. 291-302.) (Hereby incorporated by reference.) The cells were propagated at 37°C in the humid 5% $CO_2$ atmosphere. Stocks of the HIV-1 isolates HTLV-$III_B$ were acquired from the NIH AIDS Research and Reference Reagent Program (catalog no. 398), and were harvested from cultures of chronically-infected CCRF-CEM cells. For routine propagation, virus containing culture fluids were clarified of cells by low speed centrifugation and passed through 0.45 µm filters. Infectious virions were quantitated on MT-2 cells in microculture using cytopathic effect (CPE) as end point for infection. (Scudiero DA, Shoemaker RH, Paull KD, Monks A, Tierney S, Nofziger TH, Currens MJ, Seniff D, and Boyd MR: Evaluation of a soluble tetrazolim/formazan assay for cell growth and drug sensitivity in culture using human and other tumor cell lines. Cancer Res 1988;48:4827-4833.) The 50% tissue culture infectious dose ($TCID_{50}$) was calculated by the method of Reed and Muench. (Reed LJ, and Muench H: a simple method of estimating fifty percent endpoints. Amer J Hygiene 1938;27:493-497.) CCRF-CEM cells used for virus xenotransplantation were acutely infected with stock dilution of HIV-1 at a MOI (input multiciplicity of infection) of 0.1 followed by adsorbtion for 2 hours at 37°C. All HIV-1 procedures are performed within a Biosafety Level 3 (BSL-3) facility, and all procedures adhere to BSL-3 guidelines. (Centers for Disease Control (CDC): Agent summary statement for human immunodeficiency virus and report on laboratory-acquired infection with human immunodeficiency virus. MMWR 1988;37 (S-4): 11-15.)

Animals, Diet, Environment, and Cell/HIV Transplantation

As previously described, (Wetherall NT: The development of HIV-1 p24 antigenemia in the athymic "nude' mouse. In: Animal Models in AIDS. Schellekens H and Horzinek MC (eds.). Elsevier; Amsterdam, 1990, pp. 291-302. Johnson MD, Davis BW, and Wetherall NT: Production of proto-oncogene expressing control tisseus for in situ hybridization and immuno-histochemical studeis. J Environ Path Tox and Onc 1989;9:171-190.) Outbred, athymic, 27±1°C, without antibiotic coverage, in a specific-pathogen-free room under laminar-flow HEPA-filtered air. All bedding, cages, water, and other material coming in direct contact with the mice were autoclaved before use. Animals were permitted access to food and water ad libitum. The mice were fed a diet of pelleted chow which contains elevated levels of heat-sensitive nutrients (Purina autoclavable rodent laboratory chow #5010). The solid-wall and -bottom cages are covered by microisolators, and the litter was changed twice a week. The BSL-3 guidelines as outlined by the CDC were followed. (Centers for Disease Control (CDC): Agent summary statement for human immunodeficiency virus and report on laboratory-acquired infection with human immunodeficiency virus. MMWR 1988;37(S-4):11-15. Milan G, and D'Souze P: HIV infections in SCID mice: safety considerations. ASM News 1990;56:639-642.) A maximum of ten mice were housed in large cages for the experimental period.

CEM cell cultures, both HIV infected or uninfected, were harvested and washed with serum free media and reharvested. Inocula were standardized by counting the cells with a hemocytometer, and adjusting the cell suspension with serum free medium. Cells suspended in 0.2 ml media were injected subcutaneously (s.c.) into the intrascapular region using a syringe attached by luer lock to a 22ga. TEFLON pediatric angiocatheter. This catheter was first tunneled through the subcutaneous tissues to eliminate leakage of the inoculum. Aseptic procedures were used throughout.

Animals were delivered on the Monday of the initiation of the experiment, and were allowed two days to acclimate to the new environment. The next Wednesday, the animals were exposed to 500 Rad's of $^{137}Cs$ irradiation (University of Minnesota Medical School's J.L.Shepherd Mark I irradiator). This dose reduces natural killer cell activity, and usually no morbidity is apparent. On Thursday the drug dosing was started, and on Friday of this week the animals were inoculated as described above. The mice were observed the following day and at least three times a week for the during of the experiment. At each of these time points the animal group weight was determined, and the inoculation site was gently palpated to determine the date of gross tumor onset, or the tumor size which was measured in two dimensions via calipers. The tumor volume was calculated from measurements in two dimensions using the formula for a prolate ellipsoid, $\pi/6$ LW. (Graham BS, and Wetherall NT: Growth of human cell lines in BALB/c mice. Cancer Res 1990:50:5943-5946. Skalrin NT, Chahinian AP, Feuer EJ, Lahman LA, Szrajer L, and Holland JF: augmentation of activity of cis-diamminedi-chloroplatinum(II) and mitomycin C by interferon in human malignant mesothelioma xenografts in nude mice. Cancer Res 1988;48:64-67.) Significance of differences between groups was evaluated by the unpaired Student's t-test, and probability values were specified in two tails.

At the termination of an experiment, the animals were placed under Methoxyflurane anesthesia. After the pain responses were absent, the animals were bled by cardiac puncture until dead. If the animals were not dead by this means, cervical dislocation was used. These methods are consistant with the recommendations of the Panel of Euthanasia of the American Veterinary Medical Association. After death the animals were necropsied and tumor tissue was obtained aseptically for CEM cell examination and for HIV antigen expression using an indirect immunofluorescent assay. (Montefiori DC, and Mitchell WM: Infection of the HTLV-II bearing T-Cell line C3 with HTLV-III is highly permissive and lytic. Virology 1986:155:726-731.)

In conducting research using animals, the investigators adhered to the "Guide for the Care and Use of Laboratory Animals," prepared by the committee on Care and Use of Laboratory Animals of the Institute of Laboratory Animal Resources, National Research Council (NIH Publication No. 86-23, Revised 1985).

P24 Enzyme Immunoassay

The p24 enzyme immunoassay (EIA) used was the unmodified procedure commercially available from Coulter Corporation (Hialeah, F1), which uses a murine monoclonal antibody to the HIV core protein coated onto microwell strips. The assay detects p24 gag antigen in culture supernatants, plasma, and serum. Non-specific cross reactions with mouse serum are not seen with this assay.

Preparation and Administration of Compounds

Doses of KU10280 were freshly prepared for each day's drug administration. Ampules for daily injection was provided by the sponsor. Each ampule was reconstituted with 1.2 ml of sterile distilled water, and 0.1 ml was injected i.m. (intramuscular), day -1 to day 11, and s.c., day 19 to day 38 for daily drug administration, unless noted. Control animals received an injection of 0.1 ml water (placebo).

AZT was procured as over-the-counter RETROVIR$^R$ 100mg capsules. The contents from each capsule was dissolved in 200 ml of sterile distilled water by mixing on a stir plate for one hour. The inactive filler component of the capsule contents was removed by centrifugation at 3,000 rpm for 10 minutes. The supernatant was adjusted with additional sterile distilled water to achieve a final concentration of .125 mg/ml. The animals were monitored daily to assure an average consumption of 4ml/day/mouse, which was achieved. This method delivers .5 mg AZT/day/mouse or 20 mg AZT/kg/day. The prepared dilutions of the KU10280 and AZT were soluble when used, and AZT was prepared fresh on a weekly basis to maintain stability.

EXPERIMENTAL DESIGN

The in vivo assay using this model consisted of testing the KU10280 in a prophylactic manner, whereby a single dose of agent is administered to a group of mice one day prior to HIV challenge. The test and control animals were followed for a period of 39 days with washout. The specific experimental design consisted of the comparisons of the following groups:

Table 11

| | |
|---|---|
| Cell control -(Group No. 1) | 12 mice transplanted with human CCRF-CEM cells only |
| Virus control -(Group No. 2) | 12 mice transplanted with CCRF-CEM infected with HIV at a M.O.I. of 0.1, placebo treated |
| Drug control -(Group No. 3) | 6 mice treated with KU10280, at 8.3 mg/mouse/day administered i.m. or s.c. daily, transplanted with CCRF-CEM cells (without virus) |
| AZT control -(Group No. 4) | 7 mice treated with AZT (0.5 mg/day/mouse or 20mg/kg/day), challenged with HIV at the same M.O.I. as the virus control |
| Test Drug -(Group No. 5) | 6 mice treated with KU10280, at 8.3 mg/mouse/day administered i.m. or s.c. daily, challenged with HIV at the same MOI as the virus control |

Upon the experiment termination and animal sacrifice, serum p24 antigen levels were determined as the experimental endpoint.

Results

Tables 12 and 13 outline the overall course of the experiment. Due to irradiation problems (apparent overdose), morbidity was readily apparent by day 11, causing the cessation of all treatments. All mice exhibited cutaneous petechiae to varying degrees which was due to irradiation included thrombocytopenia, especially excerbated in the AZT treated group. Mortality appeared in all groups (days 11-20, with the loss of AZT treated animal the day prior to the experiment's end). An increased starting number of animals, minimizing the negative impact of the deaths. It should be noted that the percent survival was equal or better in the KU10280 treated animals suggesting some protection from the irradiation. The mice recovered from the apparent irradiation poisoning to restart treatment on day 19. The initial tumor formation occurred on day 11 (groups 1 & 3), day 15 (group 4), or day 18 (groups 2 & 5) and progressed until the termination of the experiment at day 39. The total period of drug treatment was 31 days (1 day prior to xenotransplantation through day 10, and days 19 - 39).

TABLE 12

| COURSE OF EXPERIMENT | | | |
|---|---|---|---|
| Group No. | Number of Mice at Day 0 | Number of Mice at Day 39 | % Survival |
| 1 | 12 | 7 | 58.3 |
| 2 | 13 | 9 | 69.2 |
| 3 | 6 | 6 | 100.0 |
| 4 | 7 | 3 | 42.9 |
| 5 | 6 | 4 | 66.6 |

TABLE 13

| COURSE OF EXPERIMENT (CONT.) | | | |
|---|---|---|---|
| Group No. | Treatment Ceased | Mortality No. Mice | Treatment Restarted |
| 1 | Day 11 | 3 x Day 15<br>1 x Day 19<br>1 x Day 20 | Day 19 |
| 2 | Day 11 | 1 x Day 11<br>2 x Day 15<br>1 x Day 16 | Day 19 |
| 3 | Day 11 | | Day 19 |
| 4 | Day 11 | 1 x Day 13<br>2 x Day 14<br>1 x Day 38 | Day 19 |
| 5 | Day 11 | 1 x Day 15<br>1 x Day 20 | Day 19 |
| Note: By day 11, 100% of Mice exhibited cutaneous petechaiae, indicating Thrombocytopenia due to irradiation poisoning. Mice recovered by Day 19. | | | |

At the termination of the experiment, the group body weights, group tumor volumes, and the serum p24 levels were determined, displayed in tables 14, 15, and 16. Levels of significance (p values) comparing the various groups are listed below.

TABLE 14

| TERMINAL MEASUREMENTS | | | | | | | | | | | |
|---|---|---|---|---|---|---|---|---|---|---|---|
| Mean Group Body Weight (grams) | | | | | | | | | | | |
| | MOUSE NO. | | | | | | | | | | |
| | 1 | 2 | 3 | 4 | 5 | 6 | 7 | 8 | 9 | Mean | S.D. |
| Group No. 1: | 34 | 30 | 36 | 34 | 28 | 35 | 26 | | | 32 | 3.8 |
| Group No. 2: | 24 | 21 | 19 | 25 | 23 | 20 | 18 | 18 | 22 | 21 | 2.6 |
| Group No. 3: | 28 | 25 | 35 | 27 | 34 | 23 | | | | 29 | 4.8 |
| Group No. 4: | 21 | 21 | 16 | | | | | | | 19 | 2.9 |
| Group No. 5: | 26 | 22 | 22 | 22 | | | | | | 23 | 2.0 |

TABLE 15: MEAN GROUP TUMOR VOLUME ($CM^3$)

| Group No. | Mouse 1 | Mouse 2 | Mouse 3 | Mouse 4 | Mouse 5 | Mouse 6 |
|---|---|---|---|---|---|---|
| 1: | 5.94 | 6.11 | 8.54 | 9.63 | 5.77 | 9.01 |
| 2: | 2.68 | 1.15 | 1.33 | 0.94 | 2.47 | 3.18 |
| 3: | 6.81 | 4.58 | 12.4 | 7.86 | 15.6 | 2.68 |
| 4: | 1.5 | 1.85 | 0.73 | | | |
| 5: | 1.42 | 2.28 | 0.89 | .004 | | |

| Group No. | Mouse 7 | Mouse 8 | Mouse 9 | Mean | S.D. |
|---|---|---|---|---|---|
| 1: | 3.35 | | | 6.9 | 2.24 |
| 2: | 0.53 | 2.55 | 0.70 | 1.7 | 0.99 |
| 3: | | | | 8.3 | 4.86 |
| 4: | | | | 1.4 | 0.57 |
| 5: | | | | 1.1 | 0.95 |

TABLE 16: MEAN GROUP SERUM p24 (ng/ml)

| Group No. | Mouse 1 | Mouse 2 | Mouse 3 | Mouse 4 | Mouse 5 | Mouse 6 |
|---|---|---|---|---|---|---|
| 1: | 0 | 0 | 0 | 0 | 0 | 0 |
| 2: | 1.213 | 0.022 | 1.048 | 0.978 | 0.727 | 0.07 |
| 3: | 0 | 0 | 0 | 0 | 0 | 0 |
| 4: | 0.156 | 0.280 | 0.269 | | | |
| 5: | 0.067 | 0.042 | 0.124 | 0.028 | | |

| Group No. | Mouse 7 | Mouse 8 | Mouse 9 | Mean | S.D. |
|---|---|---|---|---|---|
| 1: | 0 | | | 0 | 0 |
| 2: | 1.193 | 1.008 | 1.384 | 0.849 | 0.49 |
| 3: | | | | 0 | 0 |
| 4: | | | | 0.235 | 0.068 |
| 5: | | | | 0.065 | 0.042 |

TABLE 17

| UNPAIRED STUDENTS' t-TEST (P-VALUE) | | | |
|---|---|---|---|
| Group Comparison | Body Weight | Tumor Volume | Serum P24 Antigen |
| 1 vs 2 | <0.0001 | <0.001 | |
| 1 vs 3 | 0.2123 | 0.5041 | |
| 1 vs 4 | 0.0011 | 0.0034 | |
| 1 vs 5 | 0.0022 | 0.0010 | |
| 2 vs 3 | 0.0016 | 0.0014 | |
| 2 vs 4 | 0.3361 | 0.5673 | 0.0626 |
| 2 vs 5 | 0.2222 | 0.3544 | 0.0098 |
| 3 vs 4 | 0.0195 | 0.0480 | |
| 3 vs 5 | 0.0605 | 0.0212 | |
| 4 vs 5 | 0.1013 | 0.7506 | 0.0095 |

The serum p24 antigen at the conclusion of the HIV/CEM mouse study showed that KU 10,280 at a dose of 8 mg/day S.C. given for 28 days to six gamma irradiated nude mice transplanted with HIV-1 IIIB infected CEM cells produced a greater suppression of viral p24 greater than that from AZT 0.5 gm/kg/day. See figure 10.

Viral Blocade Studies

The mechanism of action of KU 12080 may be blocking virus from entering cells considering the following finding. The binding-inhibition profile of KU 10280 on HIV-1 IIIB was tested. Greater than 50% inhibition of adherence of whole, live HIV-1 IIIB to MT-2 cells was found at KU 10280 concentrations from 15.625 g/ml to 500 g/ml. This concentration range is in good agreement with in vitro antiviral activity of KU 10280 found using the Microtiter Infection Assay. The assay is designed to block the adherence of whole, live HIV-1 IIIB to MT-2 cells, and examines adherence under native conditions. Virus (2,500 pg p24) was incubated with KU 10280 in 250µl of growth medium for 30 minutes at 37°C. MT-2 cells (3.75 x $10^6$ in 250 µl growth medium) were added and the mixtures incubated at room temperature for two hours on a rotating platform. Unbound virus particles were removed by three washes with 5 ml of growth medium. Cell pellets were lysed in 0.5 ml of 0.5% Triton X-100, and p24 antigen quantitated by an Abbott immunoassay. Previous experiments with increasing concentrations of virus confirmed that all values were within the linear range of binding.

KU10280 did not inhibit reverse transcriptase in Triton X-100 activated IIIB lysates, it did not block syncytium formation when chronically infected H9 cells were mixed with MT-2 cells (dextran sulfate was used as a positive control), and it did not directly inactive cell-free virions. KUTAPRESSIN (Kremers-Urban Co.) injectable (minus phenol, i.e., KU 10,004) was synergistic in vitro with AZT (see figure 11).

Although the invention has been described primarily in connection with special and preferred embodiments, it will be understood that it is capable of modification without departing from the scope of the invention. The following claims are intended to cover all variations, uses, or adaptations of the invention, following, in general, the principles thereof and including such departures from the present disclosure as come within known or customary practice in the field to which the invention pertains, or as are obvious to persons skilled in the field.

SEQUENCE LISTING

(1) GENERAL INFORMATION:

(i) APPLICANT: WAGLE, SUDHAKAR S
TANAKA, S KEN
STEINBACH, THOMAS
LAWYER, CARL H
HERMANN, WILLIAM J
GAWISH, ALI ABDEL SALAM

(ii) TITLE OF INVENTION: METHOD AND COMPOSITION FOR TREATING VIRAL INFECTIONS

(iii) NUMBER OF SEQUENCES: 14

(iv) CORRESPONDENCE ADDRESS:
(A) ADDRESSEE: TILTON, FALLON, LUNGMUS & CHESTNUT
(B) STREET: 100 S. Wacker Drive, Suite 960
(C) CITY: Chicago
(D) STATE: Illinois
(E) COUNTRY: USA
(F) ZIP: 60606-4002

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US
(B) FILING DATE:
(C) CLASSIFICATION:

(vii) PRIOR APPLICATION DATA:
(A) APPLICATION NUMBER: US 07/780,084
(B) FILING DATE: 15-OCT-1991

(vii) PRIOR APPLICATION DATA:
(A) APPLICATION NUMBER: US 07/804,844
(B) FILING DATE: 04-DEC-1991

(vii) PRIOR APPLICATION DATA:
(A) APPLICATION NUMBER: US 07/728,267
(B) FILING DATE: 11-JUL-1991

(vii) PRIOR APPLICATION DATA:
(A) APPLICATION NUMBER: US 07/228,364
(B) FILING DATE: 04-AUG-1988

(viii) ATTORNEY/AGENT INFORMATION:
(A) NAME: FENTRESS, SUSAN B
(B) REGISTRATION NUMBER: 31,327
(C) REFERENCE/DOCKET NUMBER: 92050A

(ix) TELECOMMUNICATION INFORMATION:
(A) TELEPHONE: 312/456-8000

(2) INFORMATION FOR SEQ ID NO:1:

(i) SEQUENCE CHARACTERISTICS:
(A) LENGTH: 32 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:1:

```
Gly Pro His Gly Gln Ser Ile Met Leu Gly Leu Asn Ser Val Phe Tyr
1               5                   10                  15

Pro Ser Ala Ile Ile Arg Gln Ala Ala Pro Phe Phe Asp Phe Cys Trp
            20                  25                  30
```

(2) INFORMATION FOR SEQ ID NO:2:

(i) SEQUENCE CHARACTERISTICS:
(A) LENGTH: 96 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:2:

```
GGGCCGCATG GGCAAAGTAT TATGCTCGGC CTGAACAGTG TATTTTATCC AAGTGCAATA   60

ATACGTCAAG CTGCAGCTTT TTTTGACTTC TGCTGG                             96
```

(2) INFORMATION FOR SEQ ID NO:3:

(i) SEQUENCE CHARACTERISTICS:
(A) LENGTH: 110 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:3:

```
CTATAAATGT GCATTTATCA GAAGTTGATG TAAACACTAT TCTAGTACTG TTCCTTCATC   60

TAGATTGATC AATTTTAATT AAAATTAAGC ACTAAAAAAA AAAAAAAAAA             110
```

```
(2) INFORMATION FOR SEQ ID NO:4:

     (i) SEQUENCE CHARACTERISTICS:
          (A) LENGTH: 8 amino acids
          (B) TYPE: amino acid
          (C) STRANDEDNESS: unknown
          (D) TOPOLOGY: unknown

    (ii) MOLECULE TYPE: peptide

    (xi) SEQUENCE DESCRIPTION: SEQ ID NO:4:

     Tyr Lys Cys Ala Phe Ile Arg Ser
     1               5


(2) INFORMATION FOR SEQ ID NO:5:

     (i) SEQUENCE CHARACTERISTICS:
          (A) LENGTH: 25 base pairs
          (B) TYPE: nucleic acid
          (C) STRANDEDNESS: unknown
          (D) TOPOLOGY: unknown

    (ii) MOLECULE TYPE: DNA (genomic)

    (xi) SEQUENCE DESCRIPTION: SEQ ID NO:5:

CTATAAATGT GCATTTATCA GAAGT                                         25


(2) INFORMATION FOR SEQ ID NO:6:

     (i) SEQUENCE CHARACTERISTICS:
          (A) LENGTH: 21 amino acids
          (B) TYPE: amino acid
          (C) STRANDEDNESS: unknown
          (D) TOPOLOGY: unknown

    (ii) MOLECULE TYPE: peptide

    (xi) SEQUENCE DESCRIPTION: SEQ ID NO:6:

     Ile Asn Val His Leu Ser Glu Val Asp Val Asn Thr Ile Leu Val Leu
     1               5                   10                  15

     Phe Leu His Leu Asp
                 20
```

(2) INFORMATION FOR SEQ ID NO:7:

(i) SEQUENCE CHARACTERISTICS:
(A) LENGTH: 65 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:7:

```
CTATAAATGT GCATTTATCA GAAGTTGATG TAAACACTAT TCTAGTACTG TTCCTTCATC   60

TAGAT                                                               65
```

(2) INFORMATION FOR SEQ ID NO:8:

(i) SEQUENCE CHARACTERISTICS:
(A) LENGTH: 50 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:8:

```
Gly Pro His Gly Gln Ser Ile Met Leu Gly Leu Asn Ser Val Phe Tyr
1               5                   10                  15

Pro Ser Ala Ile Ile Arg Gln Ala Ala Pro Phe Phe Asp Phe Cys Trp
            20                  25                  30

Xaa Xaa Xaa Xaa Xaa Xaa Xaa Xaa Xaa Xaa Tyr Lys Cys Ala Phe Ile
        35                  40                  45

Arg Ser
    50
```

(2) INFORMATION FOR SEQ ID NO:9:

(i) SEQUENCE CHARACTERISTICS:
(A) LENGTH: 63 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:9:

```
Gly Pro His Gly Gln Ser Ile Met Leu Gly Leu Asn Ser Val Phe Tyr
1               5                   10                  15

Pro Ser Ala Ile Ile Arg Gln Ala Ala Pro Phe Phe Asp Phe Cys Trp
            20                  25                  30

Xaa Xaa Xaa Xaa Xaa Xaa Xaa Xaa Xaa Xaa Ile Asn Val His Leu Ser
        35                  40                  45

Glu Val Asp Val Asn Thr Ile Leu Val Leu Phe Leu His Leu Asp
    50                  55                  60
```

(2) INFORMATION FOR SEQ ID NO:10:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:10:

```
Xaa Glu Xaa Gly Xaa His Gly Pro His Gly
1               5                   10
```

(2) INFORMATION FOR SEQ ID NO:11:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:11:

```
Xaa Xaa Xaa Gly Thr His Xaa Pro His Gly
1               5                   10
```

(2) INFORMATION FOR SEQ ID NO:12:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:12:

```
Xaa Xaa Xaa Gly Xaa His Gly Xaa His Gly Xaa Xaa Gly Xaa Gln
1               5                   10                  15
```

(2) INFORMATION FOR SEQ ID NO:13:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:13:

```
Ala Glu His Gly Tyr His Gly Pro His Gly
1               5                   10
```

(2) INFORMATION FOR SEQ ID NO:14:

(i) SEQUENCE CHARACTERISTICS:
(A) LENGTH: 15 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:14:

CATGGICCIC ATGGI 15

## Claims

1. A substantially pure polypeptide having antiviral activity and having a molecular weight of between about 5,000 - 40,000 Daltons said polypeptide being obtained from an acetone-insoluble liver extract.

2. The polypeptide of Claim 1 in a water-soluble carrier.

3. A pharmaceutically acceptable composition comprising (a) a substantially pure polypeptide having antiviral activity and having a molecular weight of between about 5,000 - 40,000 Daltons said polypeptide being obtained from an acetone-insoluble liver extract and (b) a carrier, said polypeptide ranging from between about 5$\mu$g/ml to 500$\mu$g/ml.

4. A substantially pure polypeptide or a fragment thereof having antiviral activity, said polypeptide having at it's 5' end the amino acids shown in Sequence Id. No. 1 and its 3' end the amino acids shown is Sequence Id. No. 4.

5. A substantially pure polypeptide or a fragment thereof having antiviral activity, said polypeptide having at it's 5' end the amino acids shown in Sequence Id. No. 1 and its 3' end the amino acids shown is Sequence Id. No. 6.

6. A substantially pure polypeptide or a fragment thereof having antiviral activity, said polypeptide being comprised of amino acids at the 5' end that are coded for by DNA sequence shown in Sequence Id. No. 2 and being comprised of amino acids at the 3' end that are coded for by the DNA sequence selected from the group consisting of base pairs shown in Sequence Id. No. 3.

7. The polypeptide of Claim 6 wherein said 3' amino acid sequence is coded for by Sequence Id. No. 5.

8. The polypeptide of Claim 6 wherein said 3' amino acid sequence is coded for by Sequence Id. No. 7.

9. A therapeutic method which comprises administering to a mammal having a viral infection, which does not produce CFS, a therapeutically effective amount of the compound of Claim 1.

10. The method of Claim 1 wherein said viral infection is HIV-I.

11. The method of claim 1 wherein said viral infection is HHV-6.

12. A therapeutic method which comprises administering to a mammal having a viral infection, which does not produce CFS, a therapeutically effective amount of compound selected from the group consisting of the compounds set out in Claim 4, 5, 6, 7, and 8.

13. The method of Claim 12 wherein said viral infection is HIV-I.

14. The method of Claim 12 wherein said viral infection is HHV-6.

**15.** A synthetic peptide having the amino acid sequence shown in Sequence Id. No. 8.

**16.** A synthetic peptide having the amino acid sequence sequence shown in Sequence Id. No. 9.

**17.** A pharmaceutical composition for the treatment of an AIDS infection comprising an effective amount of AZT and the composition of Claim 1.

**18.** A pharmaceutical composition for the treatment of an AIDS infection comprising effective amounts of AZT and the composition of Claim 4.

**19.** A pharmaceutical composition for the treatment of an AIDS infection comprising effective amounts of AZT and the composition of claim 5.

**20.** A pharmaceutical composition for the treatment of an AIDS infection comprising effective amounts of AZT and the composition of Claim 6.

**21.** A therapeutic method which comprises administering to a mammal having a HHV-6 infection a therapeutically effective amount of a mammalian liver extract, the extract being characterized by being heat stable, insoluble in acetone and soluble in water.

**22.** The therapeutic method of claim 1 wherein the liver extract is a porcine liver extract.

Fig. 1

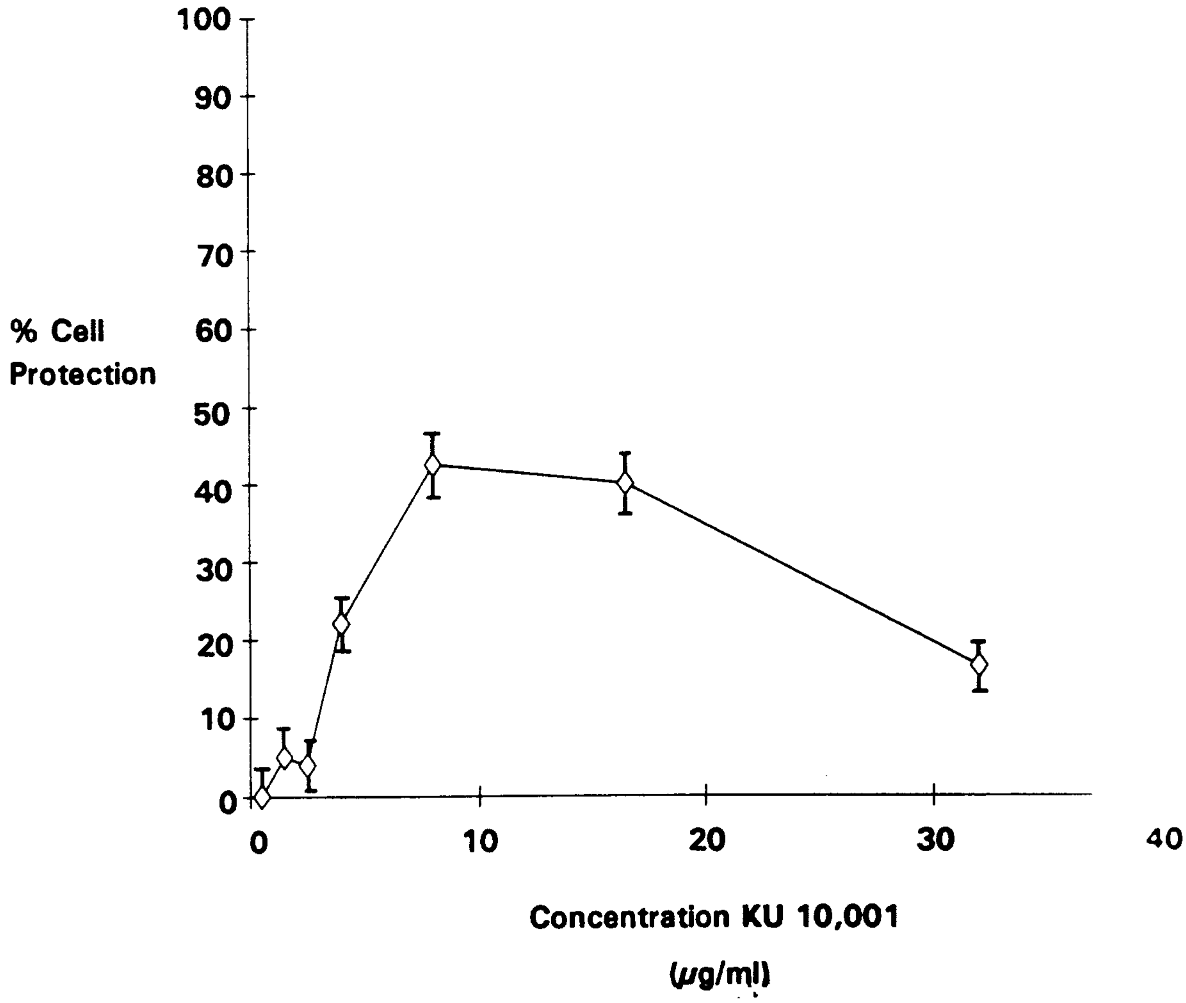
100
90
80
70
60
50
40
30
20
10
0
% Cell Protection
0
10
20
30
40
Concentration KU 10,001
(μg/ml)

# Fig. 2

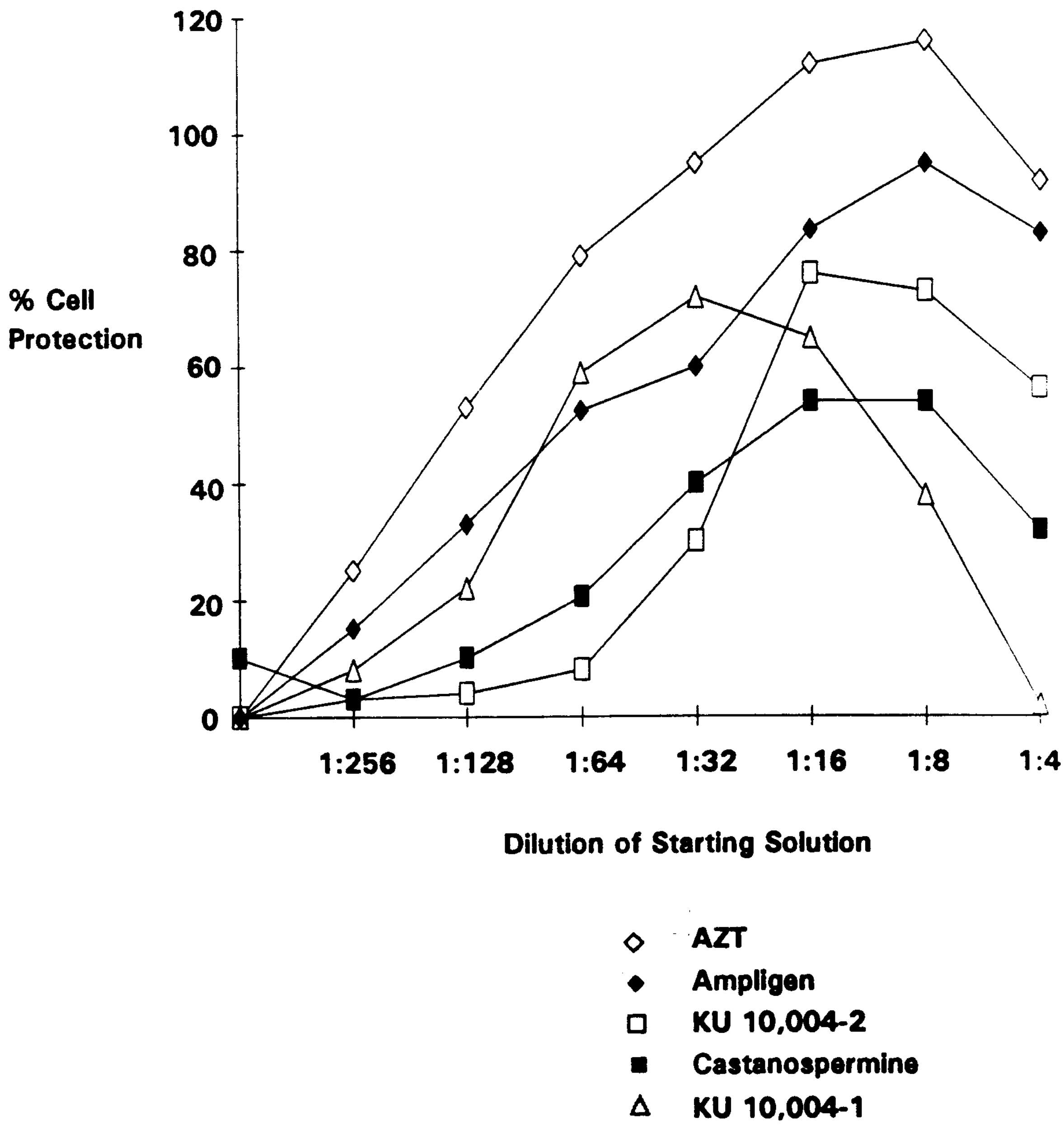
120
100
80
60
40
20
0
% Cell
Protection
1:256
1:128
1:64
1:32
1:16
1:8
1:4
Dilution of Starting Solution
AZT
Ampligen
KU 10,004-2
Castanospermine
KU 10,004-1

Fig. 3

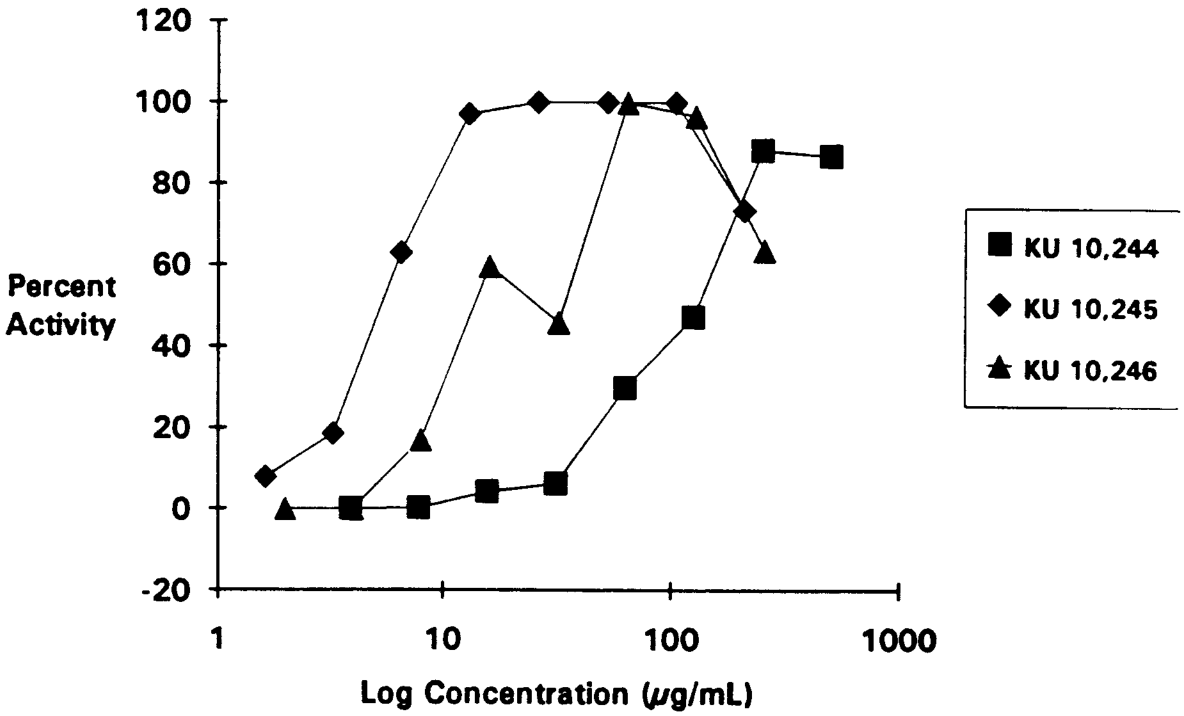
Percent Activity
120
100
80
60
40
20
0
-20
1
10
100
1000
Log Concentration (μg/mL)
KU 10,244
KU 10,245
KU 10,246

Fig. 4

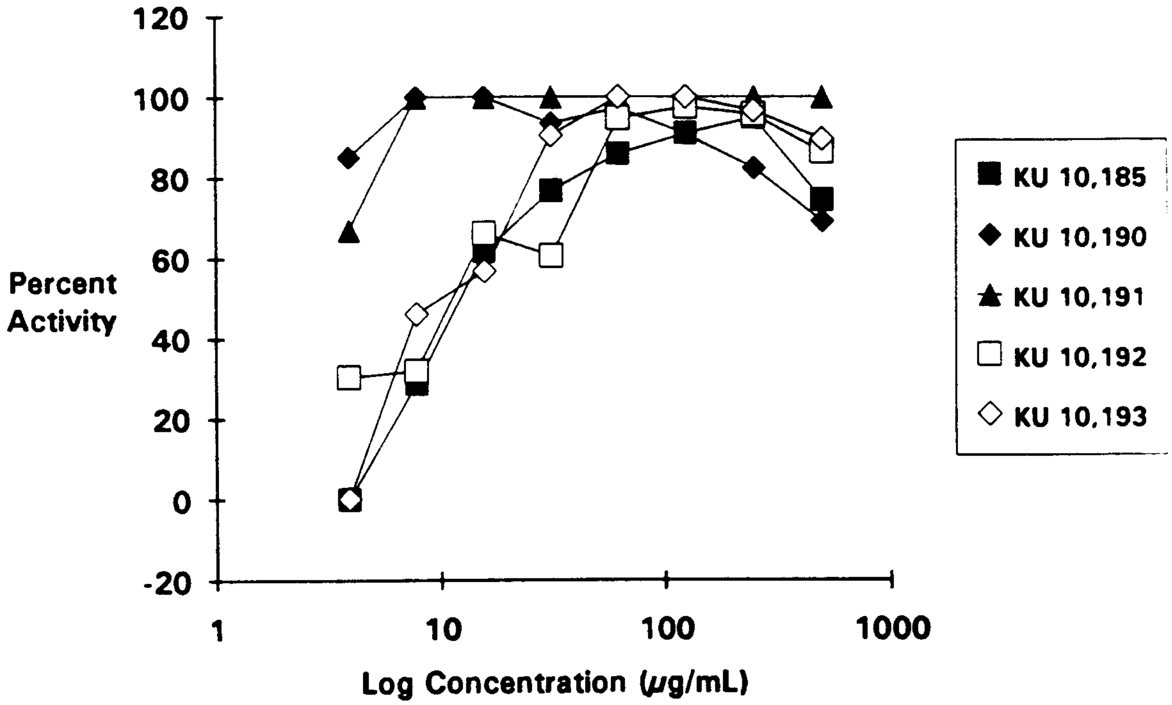
120
100
80
60
40
20
0
-20
Percent Activity
1
10
100
1000
Log Concentration (μg/mL)
KU 10,185
KU 10,190
KU 10,191
KU 10,192
KU 10,193

Fig. 5

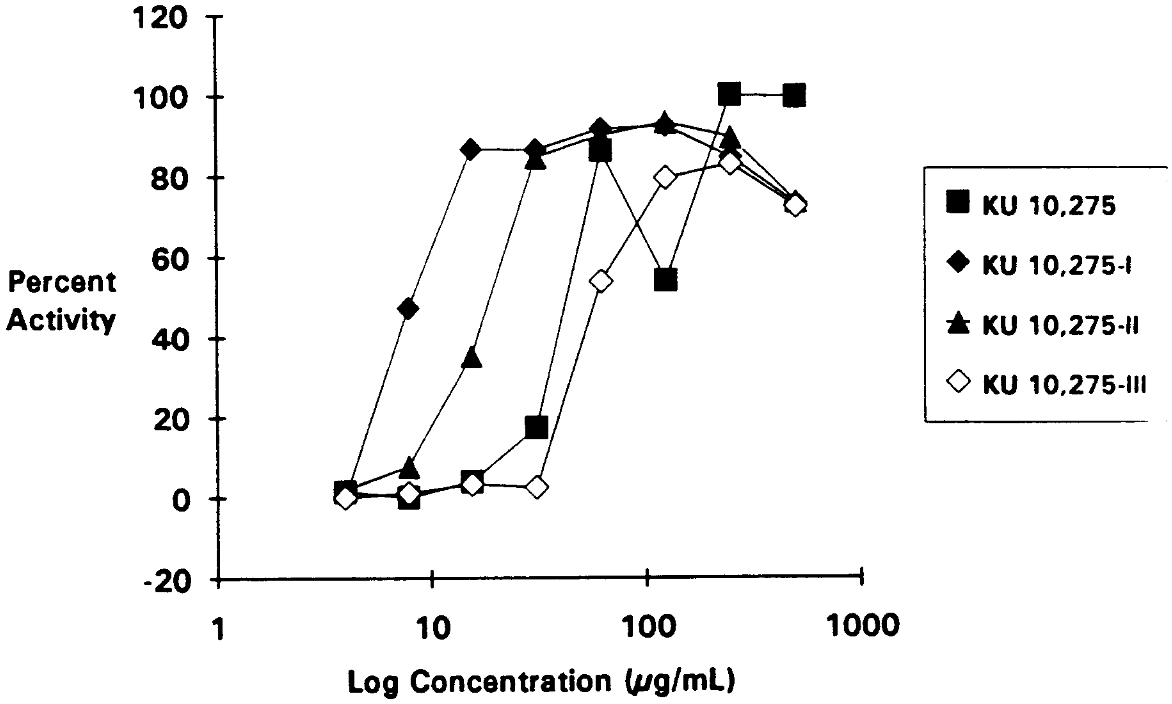
120
100
80
60
40
20
0
-20
Percent
Activity
1
10
100
1000
Log Concentration (μg/mL)
KU 10,275
KU 10,275-I
KU 10,275-II
KU 10,275-III

Fig. 6

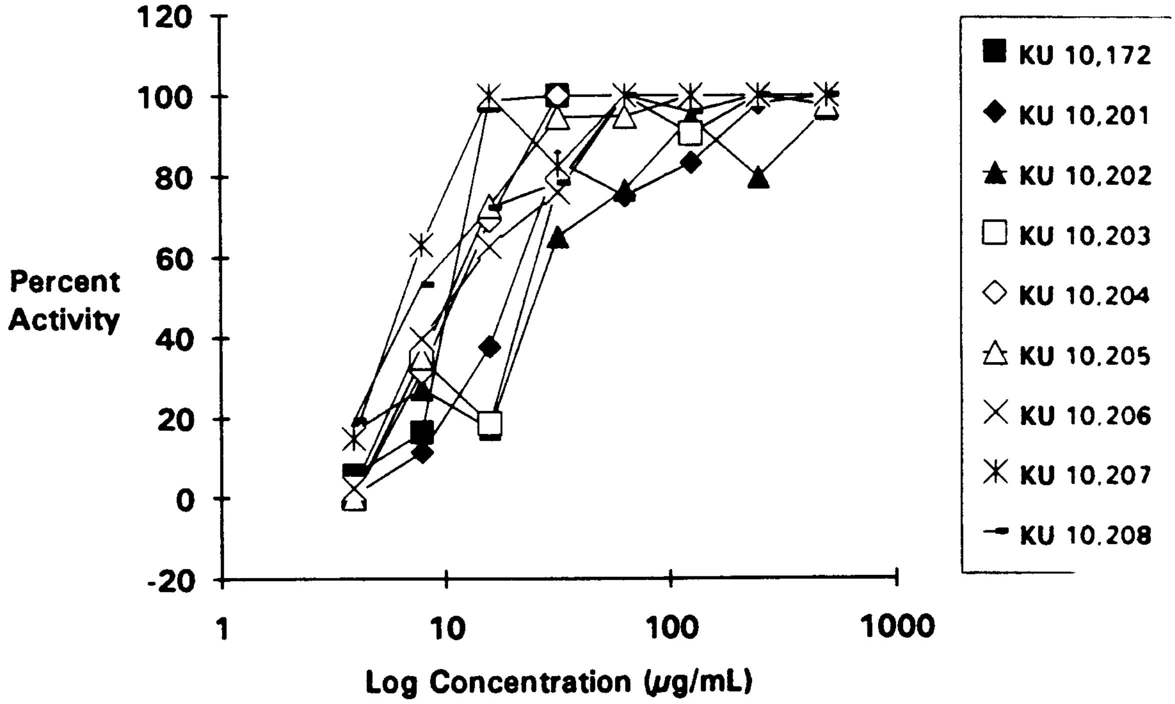
120
100
80
60
40
20
0
-20
Percent Activity
1
10
100
1000
Log Concentration (µg/mL)
KU 10,172
KU 10,201
KU 10,202
KU 10,203
KU 10,204
KU 10,205
KU 10,206
KU 10,207
KU 10,208

# Fig. 7

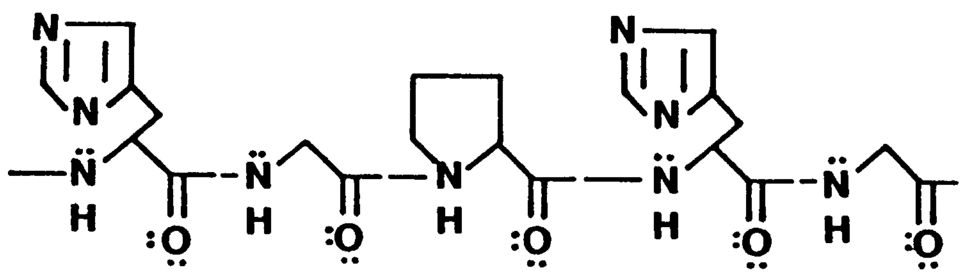

| HIS | GLY | PRO | HIS | GLY |
|---|---|---|---|---|
| CAT | GGT | CCT | CAT | GGT |
| CAC | GGC | CCC | CAC | GGC |
| | GGA | CCA | | GGA |
| | GGG | CCG | | GGG |

CODON BIAS FOR HIS
USING ALL PIG SEQUENCES
IN GENBANK 66

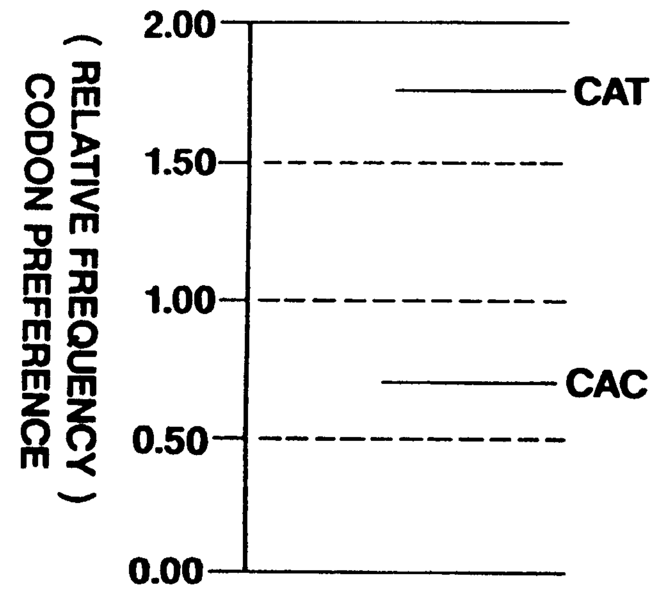

# FIG. 8A

KU214 and 215 peptide:

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

5'CATGGICCICATGGI3' PRIMER•1

5'CCTCTGAAGGTTCCAGAATCGATAG3' PRIMER•2 (CLONTECH UNI-AMP PRIMER)

Cycle 1 | denature, re-anneal

5'GGGCCGCATGGGCAAAGTATTATGCTCGGCCTGAACAGTGTATTTTATCCAAGTGCAATAATACGTCAAGCTGCACCTTT
TTTTGACTTCTGCTGG...CTATAAATGTGCATTTATCAGAAGTTGATGTAAACACTATTCTAGTACTGTTCCTTCATCTAG
ATTGATCAATTTTAATTAAAATTAAGCACTAAAAAAAAAAAAAAAAA...CTATCGATTCTGGAACCTTCAGAGG3

PRIMER•2 EXTENDS ‹-------------3'GATAGCTAAGACCTTGGAAGTCTCC5'

5'CATGGICCICATGGI3' PRIMER•1 -------------› EXTENDS

3'___CCCGGCGTACCCGTTTCATAATACGAGCCGGACTTGTCACATAAAATAGGTTCACGTTATTATGCAGTTCGACGTGG
AAAAAAACTGAAGACGACCGATATTTACACGTAAATAGTCTTCAACTACATTTGTGATAAGATCATGACAAGGAAGTAGATC
TAACTAGTTAAAATTAATTTTAATTCGTGATTTTTTTTTTTTTTTTTTGATAGCTAAGACCTTGGAAGTCTCC5'

Cycle 1 | Polymerase, dNTPs

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

Cycle 2 | denature, re-anneal

5'GGGCCGCATGGGCAAAGTATTATGCTCGGCCTGAACAGTGTATTTTATCCAAGTGCAATAATACGTCAAGCTGCACCTTT
TTTTGACTTCTGCTGG...CTATAAATGTGCATTTATCAGAAGTTGATGTAAACACTATTCTAGTACTGTTCCTTCATCTAG
ATTGATCAATTTTAATTAAAATTAAGCACTAAAAAAAAAAAAAAAAA...CTATCGATTCTGGAACCTTCAGAGG3

PRIMER•2 EXTENDS ‹-------------3'GATAGCTAAGACCTTGGAAGTCTCC5'

5'CATGGICCICATGGI3' PRIMER•1 -------------› EXTENDS

3'___CCCGGCGTACCCGTTTCATAATACGAGCCGGACTTGTCACATAAAATAGGTTCACGTTATTATGCAGTTCGACGTGG
AAAAAAACTGAAGACGACCGATATTTACACGTAAATAGTCTTCAACTACATTTGTGATAAGATCATGACAAGGAAGTAGATC
TAACTAGTTAAAATTAATTTTAATTCGTGATTTTTTTTTTTTTTTTTTGATAGCTAAGACCTTGGAAGTCTCC5
5'GGGCCGCATGGGCAAAGTATTATGCTCGGCCTGAACAGTGTATTTTATCCAAGTGCAATAATACGTCAAGCTGCACCTTT

# FIG. 8B

TTTTGACTTCTGCTGG...CTATAAATGTGCATTTATCAGAAGTTGATGTAAACACTATTCTAGTACTGTTCCTTCATCTAG
ATTGATCAATTTTAATTAAAATTAAGCACTAAAAAAAAAAAAAAAAA...CTATCGATTCTGGAACCTTCAGAGG3'
:::::::::::::::::::::::::
PRIMER•2 EXTENDS ‹------------3'GATAGCTAAGACCTTGGAAGTCTCC5

5'CATGGICCICATGGI3' PRIMER•1 -------------› EXTENDS
::::::::::::
3' ___CCCGGCGTACCCGTTTCATAATACGAGCCGGACTTGTCACATAAAATAGGTTCACGTTATTATGCAGTTCGACGTGG
AAAAAAACTGAAGACGACCGATATTTACACGTAAATAGTCTTCAACTACATTTGTGATAAGATCATGACAAGGAAGTAGATC
TAACTAGTTAAAATTAATTTTAATTCGTGATTTTTTTTTTTTTTTTTTTGATAGCTAAGACCTTGGAAGTCTCC5'

Cycle 2 | Polymerase, dNTPs
V

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

5'GGGCCGCATGGGCAAAGTATTATGCTCGGCCTGAACAGTGTATTTTATCCAAGTGCAATAATACGTCAAGCTGCACCTTTT
3'CCCGGCGTACCCGTTTCATAATACGAGCCGGACTTGTCACATAAAATAGGTTCACGTTATTATGCAGTTCGACGTGGAAAA
TTTGACTTCTGCTGG CTATAAATGTGCATTTATCAGAAGTTGATGTAAACACTATTCTAGTACTGTTCCTTCATCTAGA
AAACTGAAGACGACC···GATATTTACACGTAAATAGTCTTCAACTACATTTGTGATAAGATCATGACAAGGAAGTAGATCT
TTGATCAATTTTAATTAAAATTAAGCACTAAAAAAAAAAAAAAAAA CTATCGATTCTGGAACCTTCAGAGG3'
AACTAGTTAAAATTAATTTTAATTCGTGATTTTTTTTTTTTTTTTTT···GATAGCTAAGACCTTGGAAGTCTCC5'

further Cycles |
V

Fig. 9

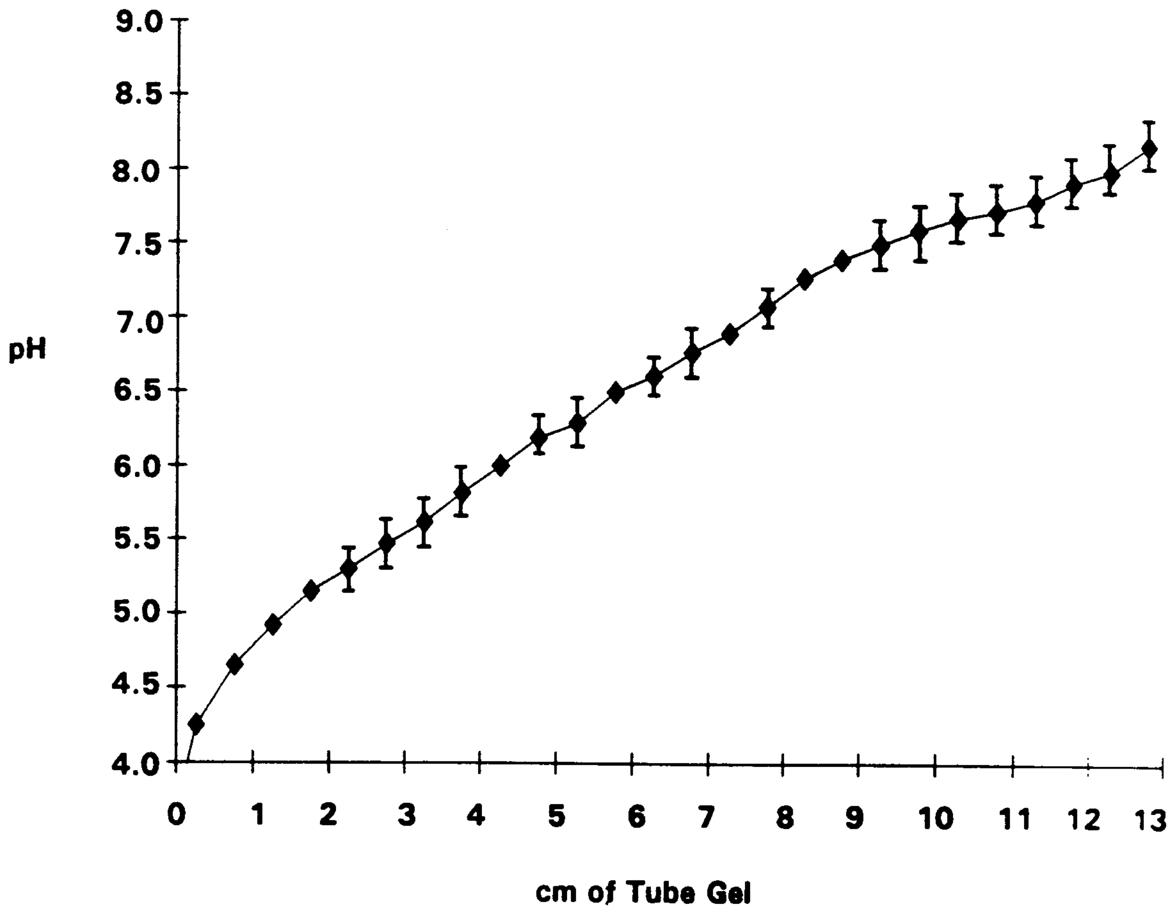
9.0
8.5
8.0
7.5
7.0
pH
6.5
6.0
5.5
5.0
4.5
4.0
0 1 2 3 4 5 6 7 8 9 10 11 12 13
cm of Tube Gel

# Fig. 10

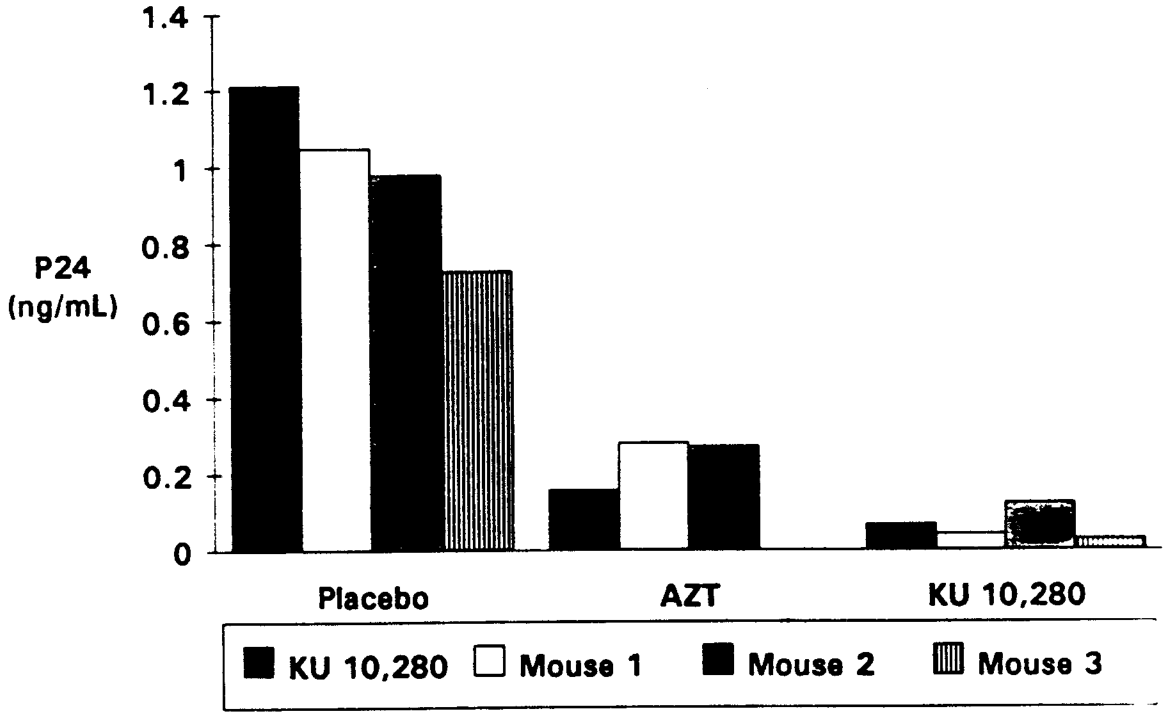
1.4
1.2
1
0.8
0.6
0.4
0.2
0
P24
(ng/mL)
Placebo
AZT
KU 10,280
KU 10,280
Mouse 1
Mouse 2
Mouse 3

Fig. 11

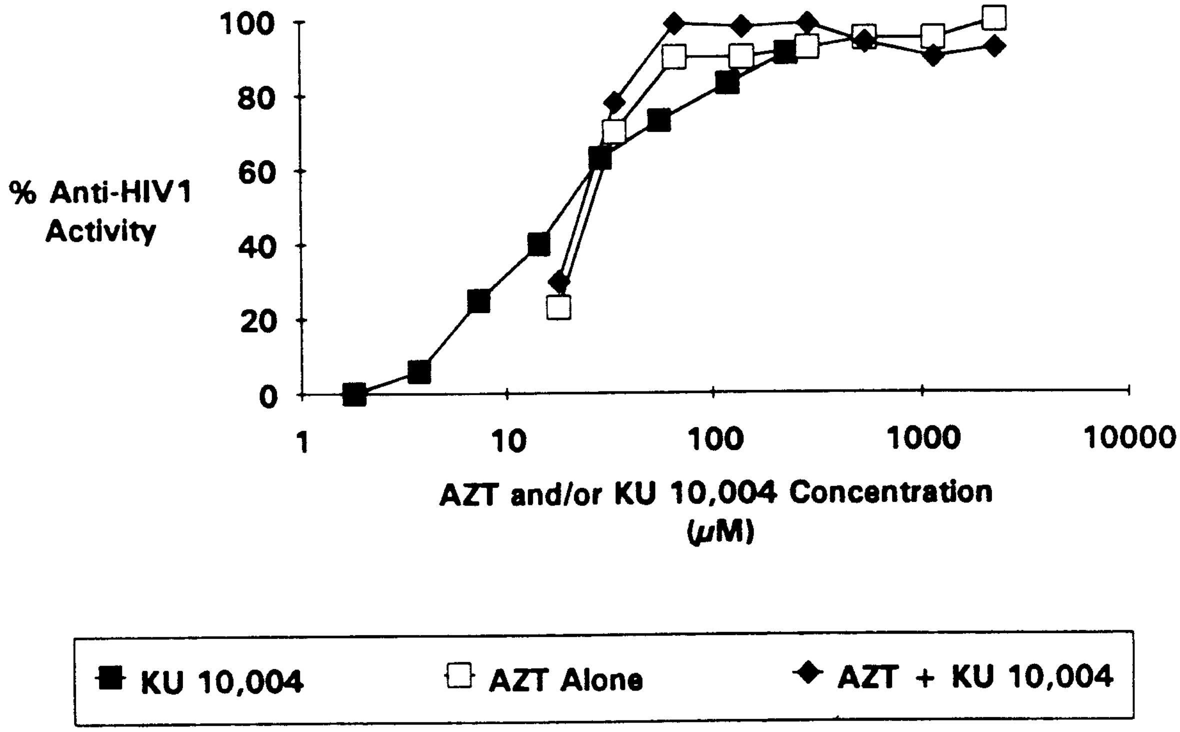
100
80
60
40
20
0
% Anti-HIV1 Activity
1
10
100
1000
10000
AZT and/or KU 10,004 Concentration (μM)
KU 10,004
AZT Alone
AZT + KU 10,004